# EUROPEAN PATENT SPECIFICATION

(11) **EP 3 684 265 B1**
(45) Date of publication and mention of the grant of the patent: **03.09.2025**
(21) Application number: 18773593.1
(22) Date of filing: 17.09.2018
(51) Int. Cl.: A61B 10/02, A61B 17/32

(54) **MEDICAL DEVICE**
MEDIZINISCHE VORRICHTUNG
DISPOSITIF MÉDICAL

(30) Priority: 18.09.2017 GB 201715002
(43) Date of publication of application: 29.07.2020
(73) Proprietor: Active Needle Technology Ltd, Abingdon, Oxfordshire OX14 3DB (GB)
(72) Inventor: IAN, Quirk, Abingdon Oxfordshire OX14 3BD (GB); SADIQ, Mumammad Rohaan, Abingdon Oxfordshire OX14 3BD (GB)
(74) Representative: Dehns
(86) International application number: PCT/GB2018/052646
(87) International publication number: WO 2019/053469

(56) References cited:
- WO-A1-2009/006291
- GB-A- 2 367 895
- US-A1- 2010 004 558

## Description

### Background

The present invention relates to a vibrating probe. In particular, the present invention relates to a vibrating needle device for use in ultrasound-guided medical procedures.

Medical procedures frequently involve the insertion of a probe into the tissue of a patient. In order to assist the medical practitioner correctly insert and position the probe, probe placement into a patient's body is often done under the guidance of ultrasound. The use of ultrasound creates a picture of the internal tissue using sound waves, assisting the clinician in guiding the probe to the tissue to be sampled. However, despite having a picture of the internal tissue, an image of the probe is often hard to reproduce due to the typically thin dimensions of the probe. Thus, accurate placement of the probe tip into the tissue is difficult, especially at steep angles and deep target locations. In addition, a relatively large amount of force is often required to insert the probe into the tissue. There is therefore a risk that the probe is bent during insertion. This may cause discomfort to the patient.

### Description of the Prior Art

GB2367895 discloses a system comprising a piezoelectric driver unit attached to the base of a needle, so that in use the piezoelectric driver imparts a longitudinal vibration to the needle enabling it to be seen by a conventional medical ultrasound imaging system. The system is designed for medical procedures such as biopsies and enables the needle to be more clearly seen on an ultrasound imaging system.

The system of GB2367895 is limited to vibrations up to 2 kHz in frequency, and does not operate in the ultrasonic range. The piezoelectric driver is offset from the base of the needle and connected to it by means of an armature, which moves in a wagging fashion and produces flexural as well as longitudinal oscillations in the needle. The device is solely concerned with visualization of the needle tip under ultrasound, and does not address the problem of reducing needle force.

US2016/0242811 discloses an ultrasonically actuated medical implement, comprising:
a first mass assembly and a second mass assembly, a channel extending along a principal axis and defined at least in part by the mass assemblies, a piezoelectric element operable to cause reciprocation between the first and second mass assemblies along a principal axis;
and a probe member received in the channel and fixedly coupled to the first mass assembly.

The probe member is typically gripped in a collet or similar mechanism.

US 2010/004558 A1 discloses a medical tool for reduce penetration force with feedback means.

WO 2009/006291 A1 discloses a medical tool for reduced penetration force.

GB 2367895 A discloses a needle location for use with an ultrasound imager.

The present invention addresses these and other limitations of the prior art.

### Summary of the Invention

According to the present invention, there is provided a device for use in a medical procedure, as claimed in claim 1.

### Detailed Description of the Preferred Embodiments

The elongate member-transducer connection may provide efficient energy transfer from the transducer to the elongate member by providing a secure connection point between the elongate member and the transducer. In addition, vibrating the elongate member reduces the amount of force require to insert the probe into body tissue during medical procedures.

The transducer may be configured to vibrate the elongate member in a lengthwise direction.

The lengthwise direction may be a longitudinal direction. Longitudinal vibration may further reduce the force require to insert the elongate member into body tissue. The amount of deflection of the elongate member upon insertion is also be reduced.

The connection arrangement may comprise a male connection member and a female connection member. The male connection member and female connection member may be configured to mate with each other. The male connection member may be on the elongate member. The female connection member may be on the transducer. The male connection member may be on the transducer. The female connection member may be on the elongate member. Corresponding male and female connection members may provide a secure connection means.

The connection arrangement may comprise a screw mechanism. A screw mechanism may provide a large point of contact between the elongate member and the transducer. This helps ensure that there is a secure and stable connection between the elongate member and the transducer. A larger point of contact also provides more reliable transmission of vibrations from the transducer to the probe. Thus there is less loss of energy, making the connection more efficient in transferring energy.

The elongate member may comprise an externally threaded portion. The transducer may comprise an internally threaded portion. The elongate member may comprise an internally threaded portion. The transducer may comprise an externally threaded portion. The threaded elongate member portion is suitably configured to mate with the threaded transducer portion. This may allow the elongate member to be screwed into the transducer for connection to the transducer. Thus the probe may be screwed into the transducer or the transducer may be screwed into the elongate member.

Alternatively, the connection arrangement may comprise a bayonet mechanism. The connection arrangement may comprise a snap-fit mechanism. Both a bayonet and snap-fit connection mechanism may provide a large, secure point of contact between the elongate member and transducer and do not rely on the provision of compression, or a gripping mechanism, to secure the probe to the transducer.

The elongate member may be connected to the transducer using a connection member. The connection member may be connected between the probe and the transducer. The connection member may be an additional, intermediate component between the elongate member and the transducer. The elongate member may be connected to the connection member using a screw mechanism. The elongate member may be connected to the connection member using a bayonet mechanism. The elongate member may be connected to the connection member using a snap-fit mechanism. The elongate member may be connected to the connection member using a clip mechanism. The transducer may be connected to the connection member using any of the aforementioned connection means.

The elongate member and transducer may be connected to the connection member using the same or different connection means.

The connection member may be a clip. The clip may comprise a first and a second gripping end. The first gripping end may be configured to attachment to the probe. The second gripping end may be configured for attachment to the transducer. The first and second gripping ends may be first and second arms. The first and second arms may be curved. The clip may be made of any suitable material, including metal or a plastics material.

The provision of an intermediate connection member may allow the elongate member and transducer to be connected together without the need to redesign either the elongate member or the transducer to allow the connection to happen. Thus any elongate member may be connected to any transducer. Thus, the transducer may be configured to vibrate any elongate member , through the use of the connection member, and a specially designed elongate member does not need to be used.

The elongate member may be a needle-like structure. The elongate member may be a needle. Needles are medical devices that are frequently used during medical procedures. The elongate member may be an ablation probe. The needle may be a biopsy needle. The needle may be a drug delivery needle. The needle may be an *in vitro* fertilization needle. The needle may be a vacuum assisted biopsy needle. The needle may be an amniocentesis needle. The needle may be a chorionic villus sampling needle. The needle may be for venous or arterial access. The needle may be for stent placement.

The needle may be a hollow needle. The hollow needle may comprise a passage configured to allow fluid to pass through the needle. This may allow the hollow needle to be used for injecting fluid into body tissue.

The transducer may comprise a channel configured to allow fluid to pass through the transducer. This allows the hollow needle to be connected to the transducer so that fluid may pass through the transducer into the hollow needle. Thus, the transducer may be used to vibrate the hollow needle during fluid injection procedures.

A tube may be configured to be inserted into a channel of the transducer. The tube may be a sterile tube. The tube may have first and second ends, the first and second ends being sealed. This may ensure that the tube environment remains free from contaminants and ensures that the inside of the tube remains sterile.

The elongate member may be configured to be attached to a first end of the sterile tube and a syringe may be configured to be attached to a second end of the sterile tube. Thus, a hollow needle may be configured to be attached to the first end of the tube. This provides a sterile environment for transferring fluid present in the syringe, through the transducer, and into the hollow needle.

The needle may be a solid needle. The solid needle may comprise a solid wire and a hollow tube that surrounds and covers the solid wire. The solid needle may comprise a stylet and a cannula. The stylet may also comprise a sample notch. The solid needle may be used to perform biopsies and the sample notch may be used to collect the tissue sample.

The system of the invention is designed to vibrate the elongate member longitudinally. When a sample notch is present, the needle also flexes at the resonance which has an advantage. Increasing the vibration amplitude can highlight the two ends of the notch under ultrasound visualization allowing the practitioner to align the sample notch with the tumour

The cannula may comprise a tip. The cannula tip may be symmetric about a central longitudinal axis of the cannula. A symmetric cannula reduces flexural motion when the needle is being vibrated by the transducer. A symmetric cannula tip is advantageous especially in embodiments in which the connection of stylet to transducer is via a screw mechanism, in which the orientation of the sample notch is unpredictable. A symmetric cannula tip allows cutting of tissue irrespective of the of position of the stylet notch.

The cannula may comprise a cutting tip. For example, the distal end of the cannula may feature a bevel, angle, or point.

The stylet may comprise a tip. The stylet tip may be symmetric about a central longitudinal axis of the stylet. A symmetric stylet reduces flexural motion when the needle is being vibrated by the transducer.

The sample notch of the solid needle may be symmetric about a central longitudinal axis of the sample notch. A symmetric sample notch may reduce flexural motion when the needle is being vibrated by the transducer.

The stylet may be configured to be connected to the transducer via the connection arrangement.

The stylet includes an integral hub. Thus, the connection member may comprise a hub. The hub may provide a convenient means of connecting the stylet to the transducer. This avoids the need for the connection mechanism to actually be present on the stylet. This may reduce the risk of damage to the stylet.

The stylet hub may comprise a base portion. Thus, the connection member may be a base portion of a stylet hub. The stylet may be configured to be attached to the base portion of the stylet hub. The stylet may be attached to the base portion of the stylet hub via a hole in the base portion of the hub. Thus, a length of the stylet may extend into the hole in the base portion of the hub. The base portion may be used to attach the stylet to the transducer. Thus, the base portion may provide a separate portion of the stylet to be connected to the transducer which may help prevent damage of the stylet as a result of the connection mechanism.

The stylet may be attached to the base portion of the stylet hub by a brazed joint. The stylet may be attached to the base portion of the stylet hub by a welded joint. The stylet may be attached to the base portion by melding. The stylet may be attached to the base portion using an adhesive. These joints may provide a secure method of attaching the stylet to the hub.

Preferably, the stylet hub comprises an externally threaded portion, extending from the base portion. The externally threaded portion of the stylet hub may be configured to engage with an internally threaded portion of the transducer. Alternatively, the stylet hub may comprise an internally threaded portion which may be configured to engage with an externally threaded portion of the transducer. This provides a simple and convenient method of attaching the stylet to the transducer.

The device may comprise a locking nut. The locking nut may be used to retain the stylet while the stylet is being connected to the transducer. Thus, the locking nut may be used to hold the stylet in place while the transducer is attached to the stylet which may help make the attachment process easier. The locking nut therefore helps the user connect the stylet to the transducer.

The locking nut preferably comprises a socket portion. The socket portion may be used to attach the locking nut to the stylet. The socket portion may be used to hold the stylet in place while the transducer is being attached to the stylet.

An internal surface of the socket portion may be shaped to correspond to an external perimeter of the base portion of the stylet hub. This ensures that the socket portion fits securely over the base portion of the stylet hub. This improves the grip that the socket portion has on the stylet hub which facilitates connecting the stylet to the transducer. The socket portion may comprise a metal insert. The metal insert may be shaped to correspond to an external perimeter of the base portion of the stylet hub to provide improved grip of the socket portion on the stylet hub.

The base portion of the stylet hub preferably has a substantially hexagonal shaped cross section. The socket portion may have a substantially hexagonal shaped cross section. Alternatively, socket portion may have a substantially octagonal shaped cross section. The socket portion may have a substantially regular polygonal shaped cross section. A polygonal cross section provides suitable grip between the base portion and the socket portion. This ensures that the stylet does not rotate while the transducer is being screwed onto the stylet, and makes the process of screwing in the stylet hub easier.

The base portion of the stylet hub and the socket portion of the locking nut are preferably configured to releasably engage. Thus the locking nut may be attached as and when needed.

The locking nut comprises an external surface. The external surface preferably comprises a grippable portion. The grippable portion may be on a portion of the external surface. Alternatively, the grippable portion may extend across the entire external surface. The grippable portion assists the user to securely grip the locking nut.

The grippable portion preferably comprises a plurality of grooves. Grooves may be formed in the locking nut at the same time the locking nut is made. Thus, a separate manufacturing step is not necessary to provide the grippable portionThe grippable portion may comprise knurling. The grippable portion may comprise any other suitable gripping mechanism.

The locking nut preferably comprises a longitudinal slit along a length of the locking nut. The longitudinal slit may extend along the entire length of the locking nut. The slit allows the locking nut to be inserted around the stylet of the needle. This permits the locking nut to be attached to the stylet without the need to disassemble the needle device, and removed in the same way.

The amplitude and/or frequency of the current supplied to the transducer may be manually controlled by a user. The user may control the voltage using a control panel. This may allow the user to adjust the voltage so that it is optimised for different types of probe. Thus, the user may ensure that the probe being used is being vibrated at or near its resonant frequency.

The device may further comprise a protective sheath. The protective sheath may be configured to enclose the transducer. The sheath may be a sterile sheath. This may ensure that the transducer does not get contaminated during medical procedures so that the transducer may be re-used.

Embodiments of the present invention will now be described, by way of example only, with reference to the accompanying drawings in which:
Figure 1 is a perspective view of a vibrating probe;
Figure 2 is a perspective view of a stylet;
Figure 3 is a perspective view of a stylet and cannula;
Figure 4 is a perspective view of a stylet and cannula;
Figure 5 is a table of cannula gauges;
Figure 6 is a table of cannula lengths;
Figures 7a-e are perspective views of cannula tips;
Figures 8a-e are perspective views of stylet tips;
Figures 9a-e are perspective views of sample notches;
Figure 10 is a perspective view of a needle housing;
Figure 11 is an exploded view of a needle housing, needle, and trigger mechanism;
Figure 12 is a cross-sectional view of a needle housing and trigger mechanism;
Figure 13 is a cross-section view of a needle housing;
Figures 14a-b are perspective views of an end cap of a needle housing;
Figures 15a-b are perspective views of a trigger lever;
Figure 16 is a perspective view of a trigger button;
Figures 17a-c are cross-section views of a trigger engaging and releasing mechanism;
Figures 18a-b are perspective views of a spring support;
Figures 19a-d are perspective views of a transducer;
Figures 20a-b are exploded views of a housing;
Figures 21a-c are views of a transducer housing;
Figures 22a-c are views of a transducer housing;
Figures 23a-d are perspective views of a front section of a transducer housing;
Figures 24a-c are perspective views of a main body of a transducer housing;
Figures 25a-b are perspective views of an end cap of a transducer housing;
Figures 26a-b are perspective views of a control box;
Figures 27a-b are perspective views of a control box;
Figure 28 is a perspective view of a foot switch;
Figures 29a-d are perspective views of a stylet hub;
Figures 30a-g are perspective views of a locking nut;
Figures 31a-h are cross-section views of a needle housing;
Figures 32a-j are cross section views of a transducer housing;

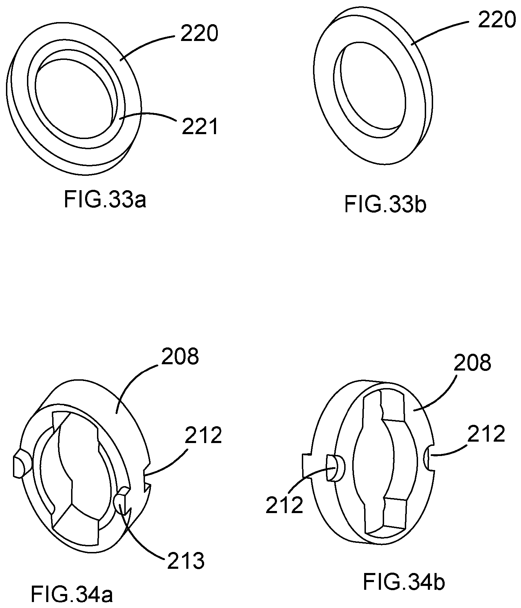

Figures 33a-b are perspective views of a spacer;
Figures 34a-b are perspective views of another spacer;
Figures 35a-e are side views of a needle housing and transducer housing;
Figures 36a-f are side views of a transducer housing;
Figure 37 is a cross section view of an alternative needle and transducer;
Figure 38 is a cross section view of an alternative transducer;
Figure 39 is a cross section view of an alternative transducer and sterile tube;
Figure 40 is a side view of an alternative transducer;
Figure 41 is a cross section view of an alternative transducer;
Figure 42 is a cross section view of an alternative needle and transducer;
Figure 43 is a perspective view of a connection member;
Figure 44 is a perspective view of an alternative embodiment of a needle housing; and
Figure 45 is a perspective view of an alternative embodiment of a spacer.

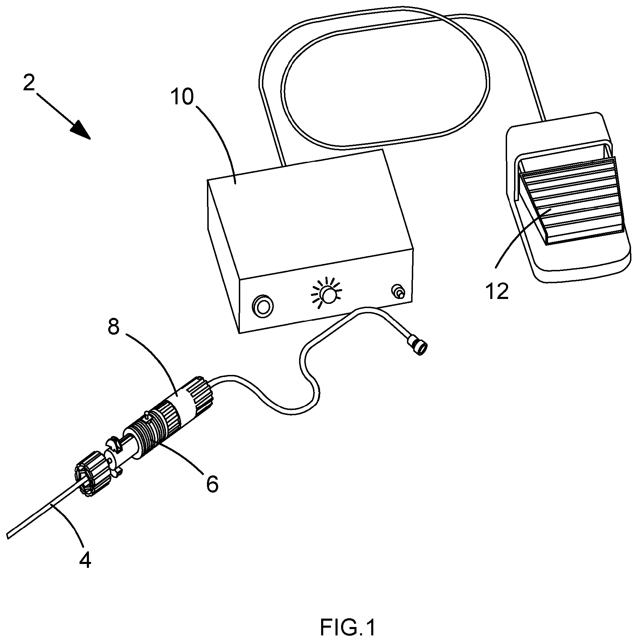

Figure 1 shows an embodiment of the inventive device 2. Here, the vibrating device 2 is a vibrating needle device 2. The device 2 comprises a needle 4 which is contained within a needle housing 6. The needle 4 is connected to a transducer 112 which is contained within a transducer housing 8. The needle housing 6 and transducer housing 8 are therefore connected together. The needle 4 is connected to an ultrasound generator unit 10 via the transducer 112. A foot switch 12 is connected to the generator unit 10 to allow the user to activate the generator unit 10. The ultrasonic generator unit 10 vibrates the needle 4 at its resonant frequency using ultrasound.

The needle 4 described herein may be used for a variety of deep tissue applications, including but not limited to kidney, liver, and lung.

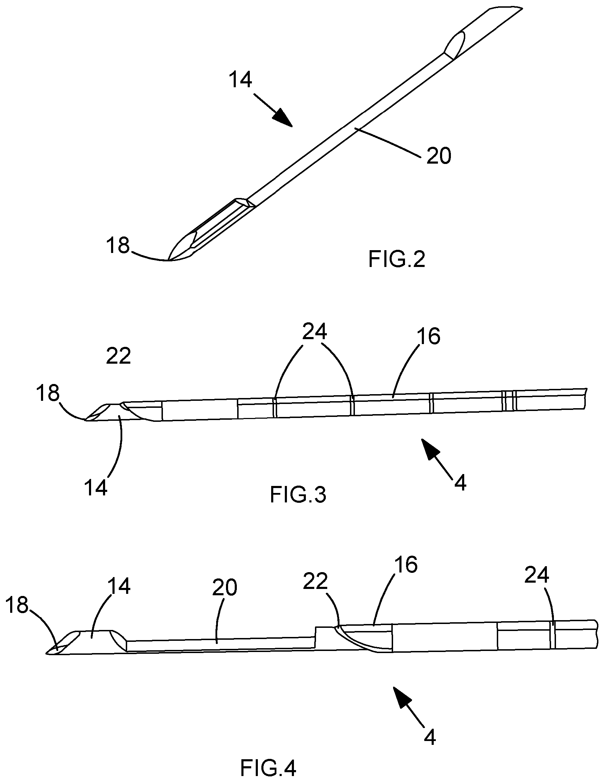

Referring to Figure 2, the needle comprises a stylet 14 and a cannula 16. The stylet 14 is a solid inner needle which comprises a cutting tip 18 at one end and a sample notch or notch 20 positioned part way along a length of the stylet 14. The tip 18 is used to cut through the layers of tissue and the sample notch 20 is used to collect the tissue sample. Referring to Figures 3 and 4, the cannula 16 is a hollow tube which has a cutting edge 22 at one end. The cannula 16 cuts the desired tissue sample to be carried in the sample notch 20. The cannula 16 surrounds the stylet 14 and is configured to be movable, relative to the stylet 14. For example, Figure 3 shows the cannula 16 in an extended configuration, in which the cannula 16 surrounds substantially the whole length of the stylet 14. Figure 4 shows the cannula 16 in a withdraw configuration, in which the cannula 16 has exposed a portion of the stylet 14, in this case the tip 18 and sample notch 20. The cannula 16 is provided with graduation markings 24 along the length of the cannula 16. The graduation markings 24 are circumferential rings equally spaced along the length of the cannula 16, however any other suitable means of providing a visual marking may be used. The graduation markings 24 provide the user with a mechanism to keep track of the depth of insertion and so are used to indicate to the user the depth of the cannula 16 inside the tissue. The graduation markings 24 are centimetre markings, however any other suitable measure could be used instead.

The vibrating needle 4 can be used to perform biopsies. The design of the needle 4, including the cannula 16 and stylet 14, varies depending on the type of biopsy procedure being undertaken. The two main types of procedure are Endcut biopsy and Trucut biopsy. Conventional Trucut biopsy requires first extending the stylet 14 from the cannula 16. The stylet 14 is then inserted, or pushed, into the tissue specimen. Whilst the stylet 14 is still inside the tissue, the cannula 16 is then slid over the stylet 14 to cut out a sample of the tissue. The tissue sample is contained within the sample notch 20 of the stylet 14. The cannula 16 and stylet 14 are then withdrawn from the tissue, the cannula 16 still covering the stylet 14.

A risk with conventional Trucut biopsy is that if the tissue specimen of interest is benign, for example the tissue feels stiffer or rubbery, the user is required to apply more than the usual amount of force to push the stylet 14 into the tissue. This may cause the tip of the stylet 14 of the needle 4 to bend or even break. A needle bending inside a patient could be painful for the patient whilst a broken needle tip could necessitate a surgical procedure to recover the broken piece of the tip.

To avoid the risk of bending or breaking the needle, the needle device 2 described herein has been designed based on the sheathed needle biopsy technique. Here, the stylet 14 is pushed into the tissue whilst the cannula 16 still covers the stylet 14. That is, the cannula 16 is not pulled back over the stylet 14 to expose the stylet 14 prior to insertion. Thus the stylet 14 and cannula 16 are inserted into tissue together and at the same time. Once inside the tissue, the cannula 16 is withdrawn by pulling the cannula 16 back so that it slides back over the stylet 14, exposing the stylet 14. The cannula 16 is then pushed forward so that it is slid back over the stylet 14 to cut out a sample of the specimen, as with the conventional Trucut technique. Both the stylet 14 and the cannula 16 are then withdrawn from the tissue.

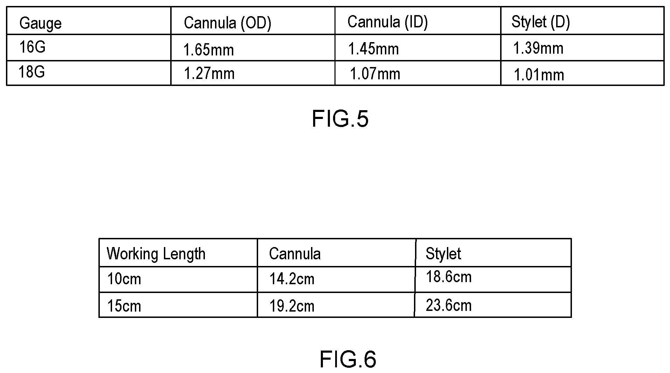

The size of the cannula 16 is determined by its gauge and length. The length of the cannula 16 represents the working length of the cannula 16 i.e. the exposed length of the cannula 16. The gauge of the cannula 16 is 16G, however it will be appreciated that any other suitable gauge may be used as illustrated in Figure 5. The gauge of the needle 4 is partly determined by the needle 4 behaviour under the influence of ultrasound vibration. In general, a thicker needle 4 is more compatible with the various modes of ultrasonic vibration. The length of the cannula 16 is 15cm, however it will be appreciated that any other suitable length may also be used as illustrated in Figure 6. The length of the cannula 16 is chosen such that it may be used with a variety of insertion depths.

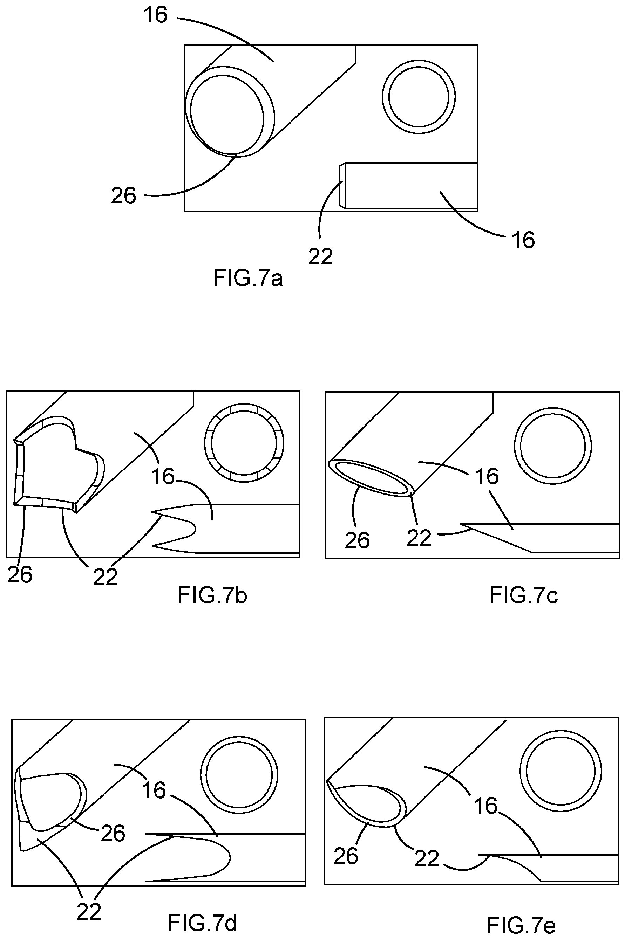

The tip 22 of the cannula has cutting edges 26 to assist in cutting the tissue sample. The orientation of the cannula tip 22 with respect to the orientation of the sample notch 20 is therefore relevant. As shown in Figure 7a, the cannula tip 22 is symmetric about a central longitudinal axis of the cannula 16, which coincides with a central longitudinal axis of the needle 4. A symmetric design is advantageous because otherwise it will be difficult for the user to ensure the alignment of the sample notch 20 with the cutting edges 26, due to the asymmetric nature of the sample notch 20 design. A symmetric design of cannula tip 22 therefore ensures that the tip design is independent of the orientation of the sample notch 20. As can be seen in Figure 7a, the cannula tip 22 has a round, tapered design. However, it will be appreciated that any other suitable tip design may be used. For example, the tip may be single-curved, double-curved, or quad-curved, as illustrated in Figure 7b-e.

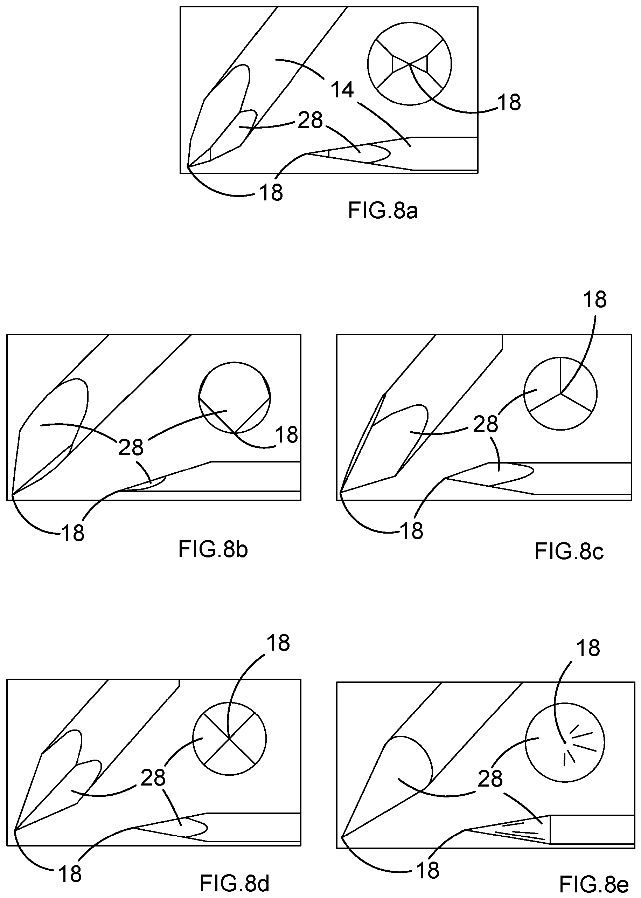

The tip 18 of the stylet of the needle 4 has a multifaceted design, as can be seen in Figure 8a. Here, the pointed tip 18 of the stylet 14 is central to the stylet 14 and needle 4. That is, the pointed tip 18 coincides with a central longitudinal axis of the style 14 and needle 4. The facets 28 are bevelled facets. The bevelled facets 28 are positioned around the central needle point 18, or stylet tip 18, in a symmetric layout. That is, the tip 18 of the needle is symmetric about a central longitudinal axis of the stylet 14, which coincides with a central longitudinal axis of the needle 4. Multiple facets provide multiple sharp edges to aid tissue cutting as the needle 4 is inserted into the tissue. A symmetric tip design is preferred as the symmetry helps reduce the production of transverse modes of vibration, which are encouraged through non-symmetries. In other embodiments, different tip designs may be used. For example the tip may be tri-bevelled, quad-bevelled, a pencil point, monofaceted, or any other suitable tip design, as shown in Figure 8b-e.

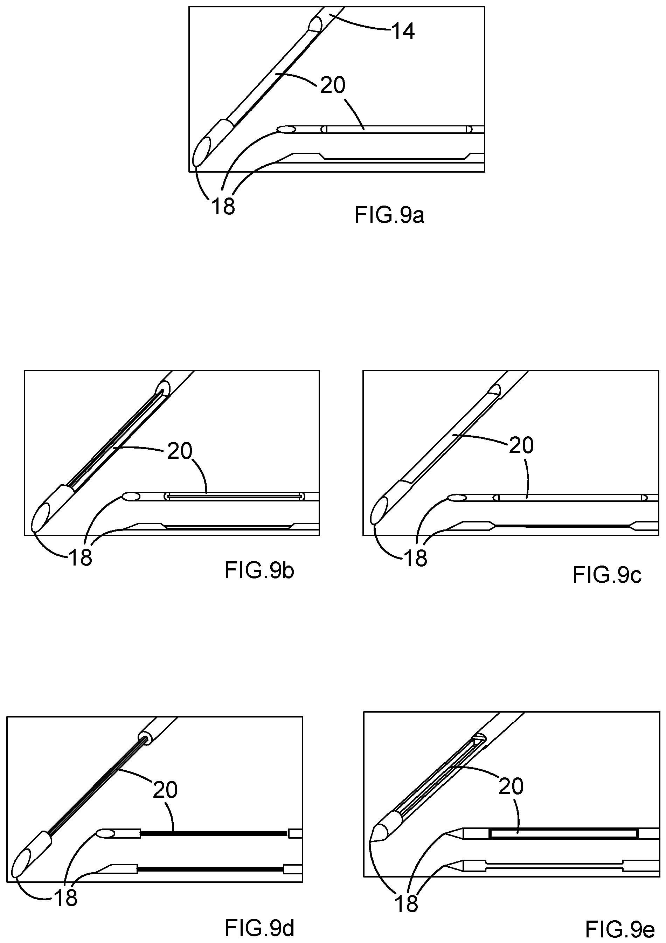

The sample notch 20 is a section of the stylet 14 in which the tissue sample is collected during the biopsy, after the tissue has been cut. The sample notch 20 is typically 20mm in length, however any other suitable length of sample notch may be used. The sample notch 20, or sample notch 20, of the needle 4 has symmetric core structure, as shown in Figure 9d. That is, the sample notch 20 is symmetric about a central longitudinal axis of the stylet 14.. The notch 20 is located part way along the stylet 14 towards the tip 18 of the stylet but spaced apart from the tip 18 of the stylet. Thus the tip 18 of the stylet and the sample notch 20 are separate from each other.

Any non-symmetry about a central, longitudinal axis of the stylet 14 leads to flexural motion at the tip of the stylet 14 thus it is important that the design of the stylet is symmetric along the entire length of the stylet. However, due to the change in the thickness of the stylet 14 before and after the notch 20, the stylet typically has a mechanically weak region which, besides having high mechanical stresses, introduces transverse modes of vibration. Both the mechanical stresses and flexural motion can lead to needle breakage during ultrasonic vibration.

A notch design which adds strength to the needle structure 4 is therefore preferred. In addition, since large sample volumes of tissue are preferred for better diagnosis, the volume of the sample notch 20 is also taken into account. Thus, to increase the strength of the needle 4, the notch 20 is coated in a high-quality surface finish (for example, having roughness value between 0.1 µm and 0.4 µm), to help avoid mechanical stresses. However, in other embodiments, the needle 4 may be polished, or electro polished, to produce a high-quality surface finish. In other embodiments, the needle 4 is cut to have a high-quality surface finish. A high-quality of surface finish is important for longevity of the needle 4 as grooves at rights angles to the length of the needle 4 can cause weak points which may lead to failure of the needle 4.

Although a core sample notch design has been chosen, it will be appreciated that many other suitable notch designs may also be used. For example, the sample notch may be a single-sided notch, a reinforced single-side notch, a double-sided notch, or a planar notch, as illustrated in Figure 9b-9e.

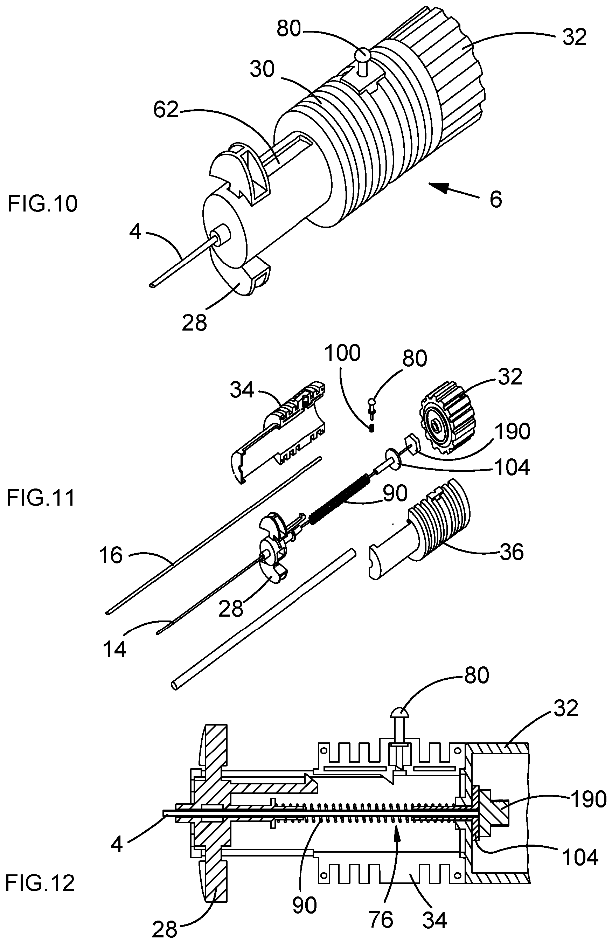

Referring to Figure 10, the needle housing 4 comprises a main body 30 and an end cap 32. The main body 30 of the housing encases the cannula 16 and stylet 14, as well as a trigger mechanism 76 for actuating insertion of the needle 4 into the tissue, allowing the tissue sample to be taken. This configuration is illustrated in Figure 12. The end cap 32 of the housing 6 connects the needle housing 6 to the transducer 112.

The main body 30 of the housing is a substantially cylindrical, hollow body. The main body of the needle housing is formed from two parts 34, 36, as shown in Figure 11. The two parts are identical to each other. Each part forms half a shell of the main body 30 of the housing. The housing body 30 is therefore made from two concave shell portions 34, 36. The two shell portions 34, 36 are joined together along their respective edges to form the substantially hollow cylindrical housing body 30, as shown in Figure 10.

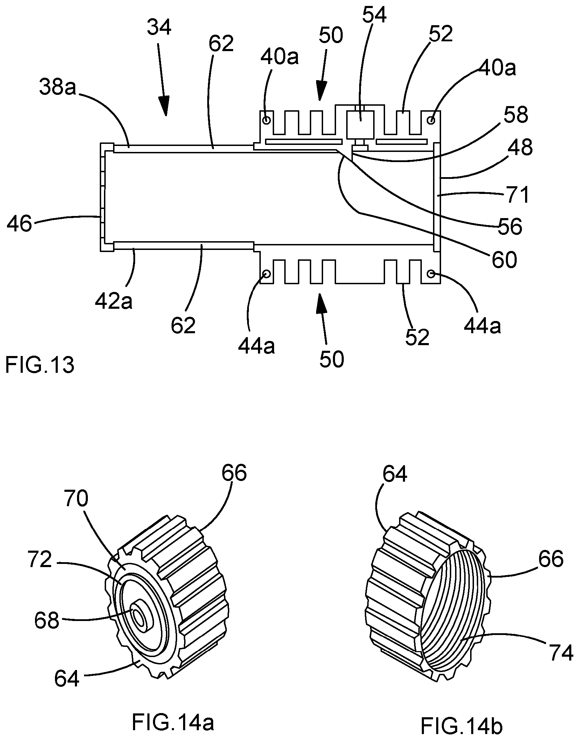

The shells 34, 36 are joined together using ultrasonic welding, although any other suitable joining process may also be used. To help with alignment of the two shell portions before they are joined together, each portion is provided with a pin and hole arrangement, as shown in Figure 13. A first side 38a of the first shell 34 comprises a plurality of pins 40a, or protrusions 40a, along an outer edge 38a while the other side 42a of the first shell 34 comprises a plurality of holes 44a along the other outer edge 42a. Corresponding second shell 36 has holes 44b along its first edge 38b and pins 40b along its second edge 42b. The holes 44b and pins 40b on the second shell portion 36 correspond to the pins 40a and holes 44a on the first shell 34. The pins 40 are inserted into the holes 44 when the two parts 34, 36 are joined together to ensure accurate alignment. The two parts, or shells, are injection moulded and made of plastic. However, any other suitable material and manufacturing process may be used.

Referring to Figure 13, the main body 30 of the housing comprises a first, or front, end 46 and a second, or rear, end 48. The rear end 48 of the main body comprises a grippable portion 50. The grippable portion 50 is a portion of the external surface of the main housing body 30 which comprises a grooved pattern to help the user grip the housing. The grooved pattern comprises a series of equally spaced apart circumferential ridges 52 which extend radially from the external surface of the housing 30. The ridges 52 are positioned at the rear end 48 of the main body and extend part way along a length of the main body 30 of the housing. Thus the ridges 52 do not extend of the whole of the external surface of the main body 30.

The body of the housing comprises a slot 54 to receive a trigger button 80. The slot 54 is positioned part way along the length of the body in-between two ridges 52. Thus, the slot 54 is positioned within the grooved pattern 50 of the main body 30. The slot 54 is configured so that the trigger button 80 extends radially through the body 30 of the housing allowing the user to actuate the trigger mechanism 76.

On an internal surface of the main body 30, substantially next to the trigger slot 54, is a trigger catch 56. The catch 56 is a protrusion which extends into the hollow portion of the main body. The catch 56 comprises a substantially flat surface 58 at one end, the flat surface 58 substantially perpendicular to a longitudinal axis of the main body 30. The catch 56 also has a sloped surface 60 which tapers towards the internal wall of the main body 30, as can be seen in Figure 13. The catch 56 is configured to engage a trigger lever 78 as will be explained in more detail later.

Towards the front end 46 of the main body 30 are slots 62 for receiving a trigger lever, as illustrated in Figure 10. As can be seen in Figures 12 and 13 there are two slots 62a, 62b substantially opposite to each other radially. The slots 62 extend longitudinally along a portion of the main body 30, terminating at the grippable portion 50 of the main body 30.

Referring to Figure 14, the end cap 32 of the needle housing 6 is substantially cylindrical having a first, or front, end 64 and a second, or rear, end 66. The front end 64 is connected to the main body 30 of the housing and the rear end 66 is connected to the transducer housing 8.

The cap 32 is a single component which has been injection moulded; however, any other suitable manufacturing process could also be used. The front end 64 of the cap is a substantially closed end having a small central passage 68 through the end potion 64. The front end 64 of the cap is ultrasonically welded to the rear end 48 of the main body of the needle housing. An alignment groove 70 and projection 72 are present on the front end 64 of the cap. The alignment projection 72 is a circumferential projection 72. The alignment groove 70 may be a circumferential groove 70. The alignment projection 72 corresponds to an alignment slot 71 on the rear end 48 of the main housing body 30. The alignment grooves 70 and projections 72 help position the end cap 32 accurately on main housing body 30.

The rear end 66 of the end cap is substantially open-ended. Thus the rear end 66 of the end cap is a hollow cylindrical portion which extends longitudinally away from the surface of the front end 64. The hollow cylindrical portion is internally threaded 74 so that it can be attached to the transducer housing 8.

The trigger mechanism 76 comprises a trigger lever 78 and a trigger button 80. The trigger mechanism 76 is configured such that it can be operated single-handed.

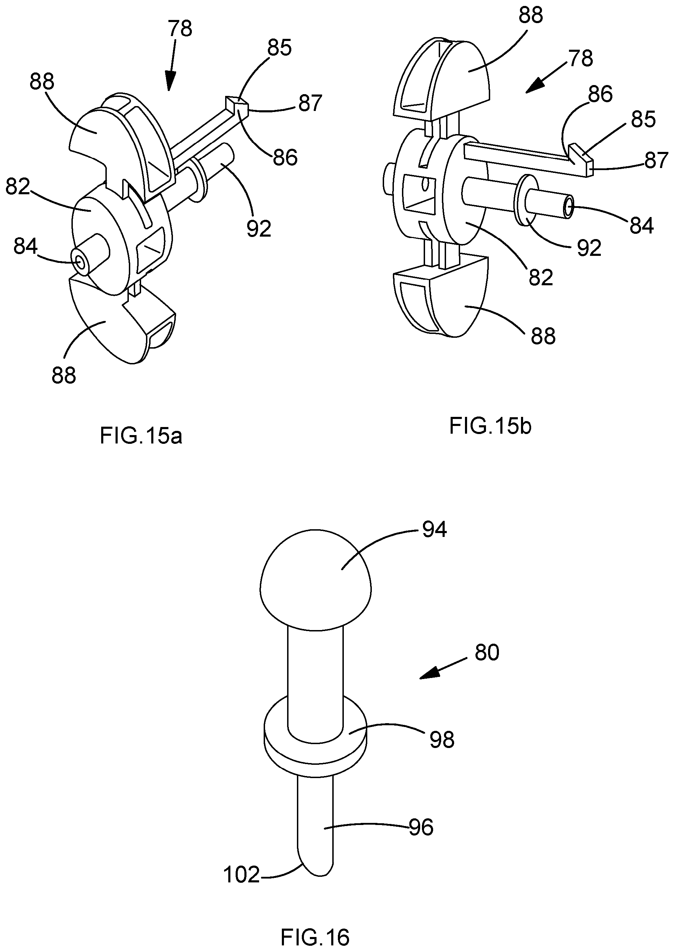

The trigger lever 78 comprises a base portion 82. The trigger lever 78 comprises a hollow passage 84 which extends through the base portion 82, as shown in Figure 15. The hollow passage 84 is in the centre of the lever. The hollow passage 84 allows the stylet 14 to be passed through the trigger lever 78, as can be seen in Figures 11 and 12, so that the trigger lever 78 can move relative to the stylet 14. The hollow passage 84 is also configured to receive an end of the cannula 16. The cannula 16 is positioned around the stylet 14, inside the hollow passage 84. The cannula 16 is attached to the inside of the hollow passage 84 so that the cannula 216 is attached to the trigger lever 78. The cannula 16 is attached to the lever 78 via a suitable UV-cured adhesive. However, any other suitable method of securely attaching the cannula could be used. Attaching the cannula 16 to the trigger lever 78 ensures that the cannula 16 moves when the trigger lever 78 moves. This also allows both the trigger lever 78 and the cannula 16 to move relative to the stylet 14.

The trigger lever 78 comprises a lever catch 86 which extends longitudinally from the base portion 82. The lever catch 86 is configured to correspond to the trigger catch 56 inside the main body 30 of the needle housing 6. Thus, the lever catch has a front sloping, or angled, surface 85 and a rear flat portion 87.The lever catch 86 and the trigger catch 56 are releasably coupled using a snap-fit connection.

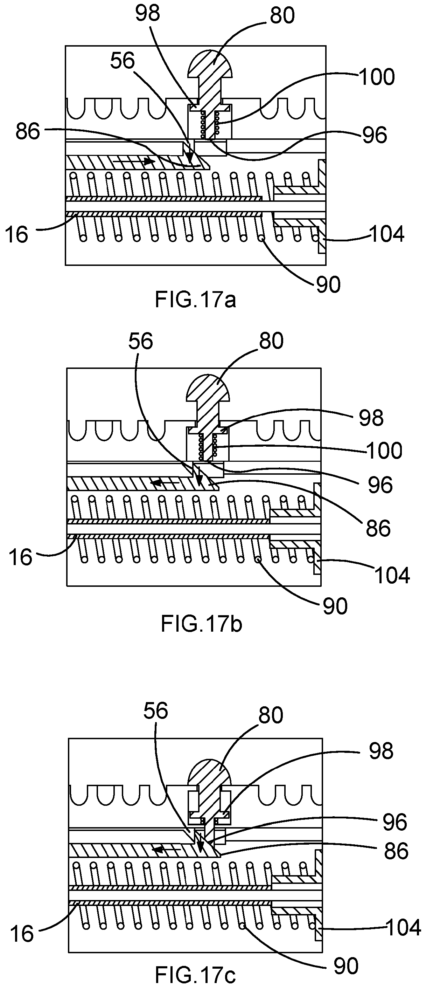

The trigger lever 78 comprises a plurality of buttons 88, or panels 88, positioned on either side of the base portion 82. As can be seen in Figure 15 the lever 78 has two panels 88 positioned substantially opposite each other radially. The panels 88 allow the user to pull the trigger lever 78 back, against a primary spring 90, until the lever catch 86 has engaged with the trigger catch 56 in the main body 30 of the needle housing, as shown in Figures 17a and 17b. Pulling the trigger lever 78 back causes the cannula 16 to be pulled back over the stylet 14, exposing the stylet 14. When the trigger 78 is subsequently released, the cannula 16 will be pushed forwards back over the stylet 14. The primary spring 90 is connected to the trigger lever 78 using a spring support 92. The spring support 92 is a longitudinally extending portion which extends from the base portion 82 of the trigger lever 78. The spring support 92 passes through the centre of the spring 90 to support the spring 90.

Referring to Figure 16, the trigger button 80 comprises a rounded end 94 and an engaging end 96, the two end portions being substantially opposite each other. Between the two end portions is a radially extending flange 98. The trigger button 80 is positioned within the trigger button slot 54 of the main housing body 30 and the rounded end 94 is configured to protrude from the main housing body 30, through the trigger button slot 54, as shown in Figure 12. A secondary spring 100 is positioned around the trigger button 80 inside the trigger slot 54. The trigger button flange 98 rests on top of the secondary spring 100. The secondary spring 100 biases the trigger button 80 so that it protrudes from the needle housing 6. The flange 98 acts as a stopper and prevents the secondary spring 100 from forcing the trigger button 80 out of the trigger slot 54. The user presses the trigger button 80, against the biasing force of the secondary spring 100, so that the engaging portion 96 extends into the internal portion of the main housing body 30.

When the trigger button 80 has been depressed, the engaging end 96 is configured to interact with the lever catch 86 on the trigger lever 78. The engaging end 96 comprises an angled surface 102 which is configured to interact with the angled surface 85 of the lever catch 86. When the engaging end 96 is forced into the main housing body 30 through the action of the user pressing the trigger button 80, the angled surface of the trigger button 102 presses on the angled surface of the lever catch 85, as shown in Figure 17c. This forces the lever catch 86 to bend radially towards the internal portion of the main housing body 30 so that the lever catch 86 and trigger catch 56 disengage. Once the two catches are disengaged, the trigger lever 78 is released. The action of the primary spring 90 then forces the trigger lever 78 towards the front end 46 of the needle housing, which in turn moves the cannula 16 forwards.

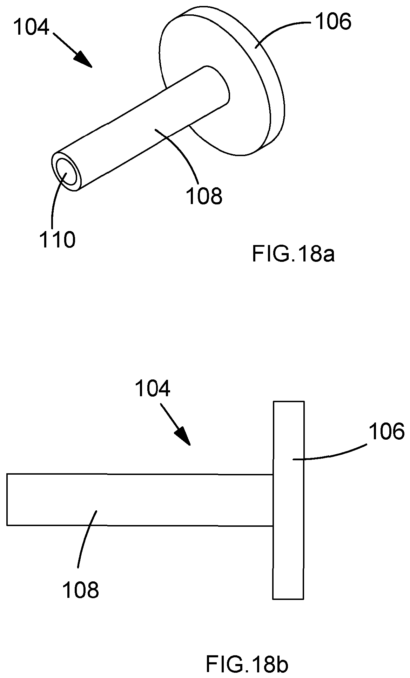

A second spring support 104 is configured to hold one end of the primary spring 90 at the rear end 48 of the needle housing while the other end of the spring 90, support by the trigger spring support 92 at the front end 46 of the housing, is compressed and released during the trigger/release operation. Referring to Figure 18, the second spring support 104 comprises a substantially flat base 106 from which a supporting portion 108 extends. The end of the spring 90 is configured to be inserted over the spring supporting portion 108. A hollow passage 110 passes centrally through the spring support 104 to allow the stylet 14 to pass through the spring support 104.

The flat base 106 of the spring support 104 is contained within the end cap 32 of the needle housing. The spring support 104 extends through the passage 68, or hole 68, in the front face 64 of the end cap. The spring support 104 stops the primary spring 90 from bending during the lever cocking process.

The primary 90 and secondary springs 100 are compression springs. The wire thickness and dimensions of the primary spring 90 are chosen in particular so that it replicates the stiffness constant of the compression spring used in conventional biopsy needles. For the secondary spring 100, the dimensions are chosen so that the spring easily fits into the needle housing trigger slot 54 and allows the user to gently push the trigger lever 78 out of the trigger catch 56. The springs are made from stainless steel, although any other suitable metal may be used.

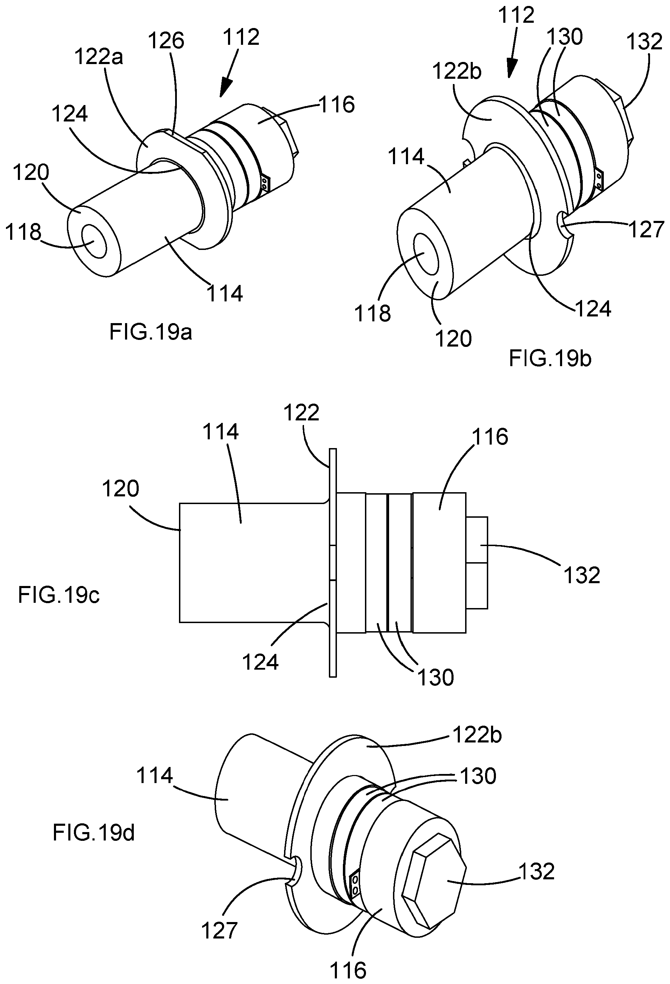

Referring to Figure 19, the transducer 112 is a standard Langevin sandwich piezoelectric transducer. The transducer 112 is configured to resonate the stylet 14 in a longitudinal mode, or direction, with an amplitude of ≤ 2µm at a frequency in the range 40 kHz to 60 kHz. This frequency range provides a balance between transducer size and large vibration amplitude. The resonant, or driven, frequency of the needle device is 46 kHz. This is defined by the frequency of the stylet 14 at which the longitudinal vibration mode is achieved. Pure longitudinal modes are preferred. This is because any asymmetry present in the stylet, especially at the notch area, produces mode coupling between longitudinal and flexural modes.

The user controls the amplitude of vibration using the ultrasound generator unit 10. However, the maximum amplitude of vibration is limited to ≤ 2µm to avoid unnecessarily large vibrations in the needle structure and to meet the condition f_{D} ≤ PRF/2 of the conventional ultrasound imaging system, where f_{D} is the Doppler shift frequency and PRF is the pulse repetition frequency. When this condition is met, the aliasing effect on the Doppler ultrasound can be avoided.

The Doppler shift frequency depends on the resonant frequency of the transducer 112, the vibration velocity at the tip of the needle 4, and the needle insertion angle or insonation angle. The pulse repetition frequency is an ultrasound system specific parameter, typically 10 kHz. If the Doppler shift frequency is higher than half of the pulse repetition frequency value then aliasing, an artefact, occurs on Doppler ultrasound which can affect the accuracy of tip visibility.

The transducer 112 comprises a front mass 114 and a back mass 116. The front mass 114 is a hollow cylinder having a passage 118 passing through a portion of the front mass 114. The passage 118 in the front mass 114 is internally threaded at a front end 120 to allow the needle 4 to be attached to the transducer 112. The front mass 114 comprises a flange 122 which extends radially away from the front mass 114. The flange 122 is positioned at a rear end 124 of the front mass 114, opposite to the front end 120 at which the needle is attached, as shown in Figure 19c. The flange 122 comprises two flat portions 126 on the perimeter of the flange 122, as shown in Figure 19a. The flat portions 126 are anti-rotation portions to prevent the transducer 112 from rotating during the needle attachment process. That is, when the needle hub is being screwed into the front mass of the transducer, the transducer will be prevented from rotating during the screw tightening by way of the flat anti-rotation portions. The front mass 114 is made of aluminium, although any other suitable metal could also be used. Although flat portions have been used as anti-rotation portions, it will be appreciated that other anti-rotation means could be used. For example, the transducer flange may comprise a plurality of spaced apart grooves 127, as shown in Figures 19b and 19d which may be configured to interact with a plurality of spaced apart protrusions. The grooves and protrusion may interact so that the transducer is prevented from rotating.

The back mass 116 is a hollow cylinder which is configured to dampen the ultrasound energy propagating toward it, resulting in large vibrations at the front mass. The back mass 116 is made of steel, although any other suitable metal could be used.

Positioned between the front and back masses is a plurality of piezoelectric rings 130. As can be seen in Figure 19c, two piezoelectric rings 130 are stacked between the front 114 and back 116 masses. The piezoelectric rings 130 are made from a high Q piezoelectric material for example Navy Type I (PZT 4) or Navy Type III (PZT 8), which are lead based piezo ceramic materials. Lead based piezo ceramics are used for low frequency, high power applications due to their lower losses and high coupling coefficient. However, the piezoelectric rings could be made from any other suitable material instead. For example, they could be made using lead-free piezo ceramics.

On each side of the piezoelectric ring 130 there is an electrode to allow for an electrical wire connection. The electrodes are brass, however any other suitable metal could be used. The two electrodes are 180° to each other and are positioned perpendicular to the anti-rotation features on the flange. This allows for easy assembly of the transducer 112 in its housing 8.

The transducer 112 further comprises a bolt 132, as shown in Figure 19c. The bolt 132 passes through the back mass 116, the stack of piezoelectric rings 130, and terminates in the front mass 114. The transducer also comprises an alumina insulator (not shown) for patient safety. The bolt 132 is a pre-stress bolt and is configured to keep the transducer assembly intact and under compression at all times to avoid the generation of cracks in the piezoelectric material during the high drive cycle. The bolt 132 is made from stainless steel, although any other suitable material could also be used. The head 134 of the bolt is hex-shaped, however any other suitable shape could be used.

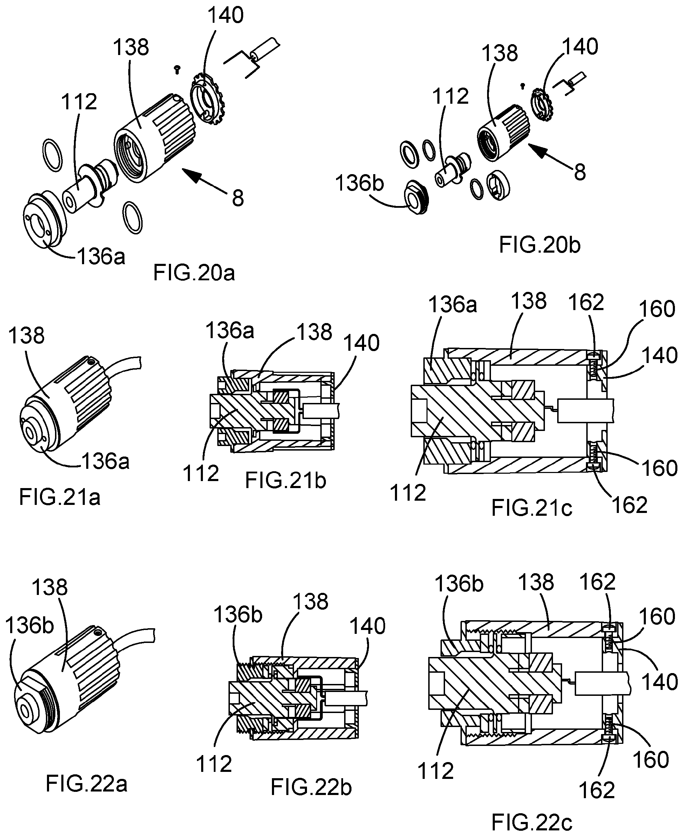

Referring to Figures 20, 21, and 22, the transducer housing 8 is used to connect the transducer to the needle housing 8 and the ultrasound generator 10. The transducer housing 8 comprises a front section 136, a main body section 138, and an end cap 140. The front section 136 of the transducer housing is connected to the end cap 32 of the needle housing and the main body section 138 of the transducer housing. The main body 138 of the transducer housing contains the transducer 112 and is connected between the front section 136 and end cap 140. The end cap 140 is used to connect the transducer 112 to the ultrasound generator unit 10.

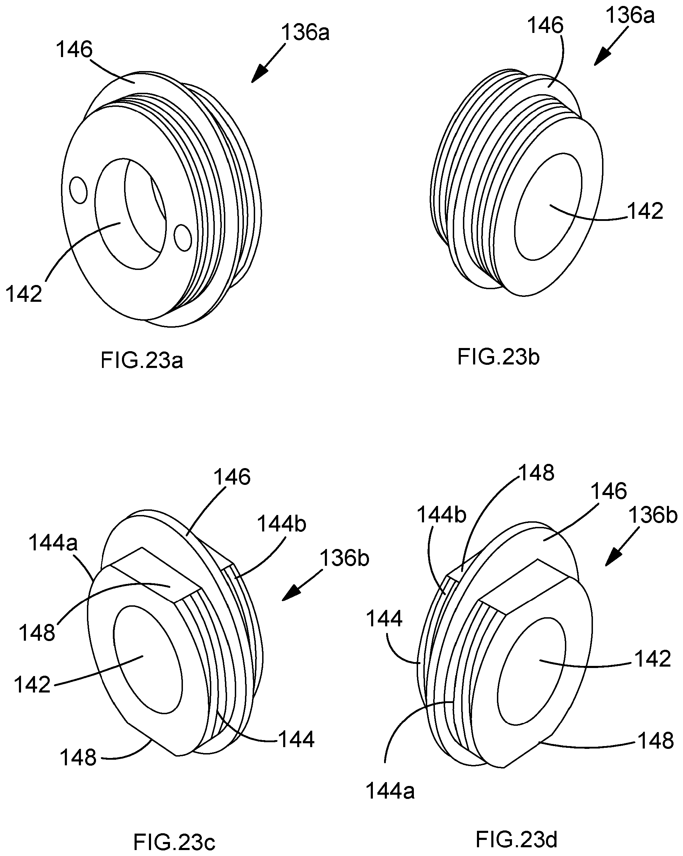

The front section 136 of the transducer housing is shown in Figure 23 and is a generally cylindrical component. The front section 136 is hollow, so that there is a passage 142 passing through the front section 136. The external surface of the front section is threaded 144 to allow the front section 136 to be connected to other components. The screw thread 144a at one end of the front section corresponds to the internal screw thread 74 on the end cap 32 of the needle housing so that these two parts can be screwed together for releasable attachment to each other. The screw thread 144b at the other end of the front section 136 corresponds to a thread of the main body 138 of the transducer housing so that these two parts can be screwed together for releasable attachment to each other.

In some embodiments, instead of being externally threaded, the front section 136 can be clipped into the main body 138 of the transducer housing and the end cap of the needle housing. For example, snap-fit connections may be present on the front section, main body of the transducer housing and the end cap of the needle housing. Other suitable connection means may also be used, for example a bayonet connection.

The front section 136 comprises a flange 146 which extends radially from the outer surface of the front section 136. The flange 146 is positioned approximately half way along the length of the front section 136, dividing the external threaded portion 144 into two separate sections 144a, 144b. The flange 146 prevents the front section 136 from being screwed too far into its connecting parts. It is therefore not possible to screw the front section 136 too far into either the main body 138 of the transducer housing or too far into the end cap 32 of the needle housing 6.

The external surface of the front section 136 further comprises first and second flat portions 148, as shown in Figures 23c and 23d. These portions are spanner flats which allow the front section 136 to be tightly screwed into its neighbouring components through the use of a spanner. The two flat sections 148 are positioned substantially opposite each other radially and at both ends of the front section 136. Embodiments in which the front section 136 does not screw into the main body of the transducer housing 8 may not have the flat sections present as they are not needed.

The front section 136 is formed from a single component by injection moulding, although any other suitable manufacturing process could also be used. The front section 136 is plastic, although any other suitable material could be used.

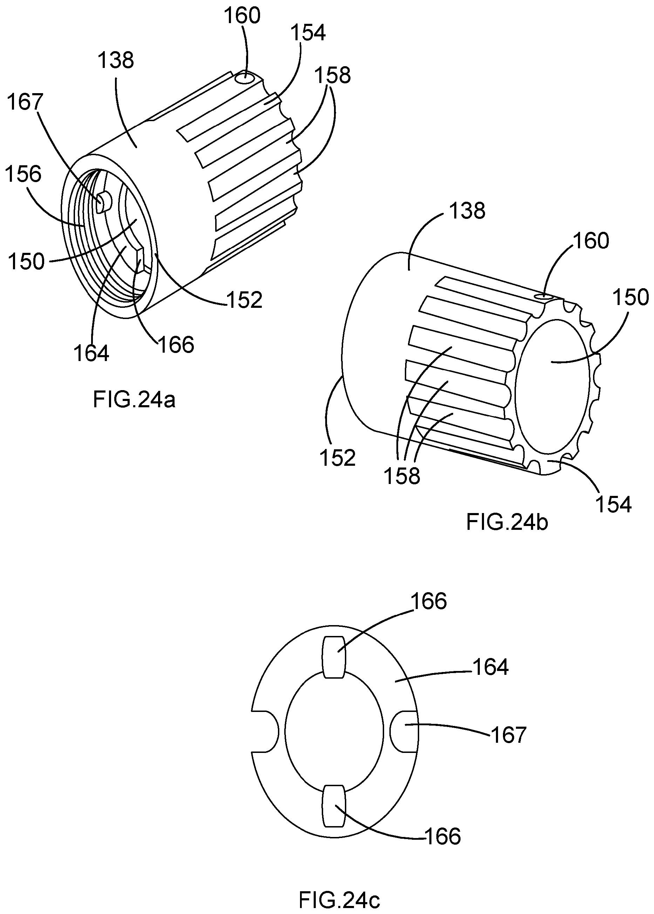

The main body 138 of the transducer housing is generally cylindrical in shape, as can be seen in Figure 24a and 24b. The main body 138 is hollow so that there is a passage 150 extending through the main body 138 between two ends 152, 154 of the main body. The diameter of the main body at one end 152 is slightly larger than the diameter of the main body at the opposite end 154. This means that the main body 138 is slightly tapered from one end to the other end, giving it a slightly conical shape. The internal surface of the slightly larger end 152 is internally threaded 156 so that the main body 136 can be connected to a neighbouring component. The internal thread 156 corresponds to the externally threaded portion 144 of the front section 136 of the transducer housing 8 so that these two components can be screwed together.

The external surface of the smaller end of the main body comprises a plurality of spaced apart grooves 158. The grooves extend longitudinally from the small end 154 of the main body 138 to approximately half way down the length of the main body 138. The grooves 158 are positioned around the entire circumference of the small end 154, as can be seen in Figure 24a and 24b. The grooves 158 provide a grippable surface to help the user grip the main body 138 of the transducer housing. It will be appreciated that any other suitable pattern for providing grip can be used, for example a plurality of raised ridges instead of grooves, or a plurality of spaced apart bumps.

At the small end 154 of the main body are holes 160 for receiving screws 162. Two holes 160 are provided, although any other suitable number of screw holes could also be used. The screw holes 160 are equally spaced about the circumference of the small end 154 of the main body 138. As can be seen in Figure 21c and 22c the two holes 160 are positioned substantially opposite each other. The screw holes 160 are used to connect the end cap 140 of the transducer housing 8 to the main body 138 of the transducer housing 8.

Inside the main body 138 of the housing is an internal, radially extending flange 164. The internal flange 164 is located part way along the length of the main body 138, towards the large end 152 of the main body 138. The flange 164 comprises a plurality of support slots 166 to help support the transducer 122 inside the transducer housing 98. The flange further comprises anti-rotation pins 167 which are configured to correspond to the anti-rotation grooves 127 in the transducer. The structure of the flange can be more clearly seen in Figure 24c.

The main body 138 of the transducer housing 8 is a single component formed via injection moulding, although any other suitable manufacturing process could also be used. The main body 138 is made from plastic, although any other suitable material could also be used.

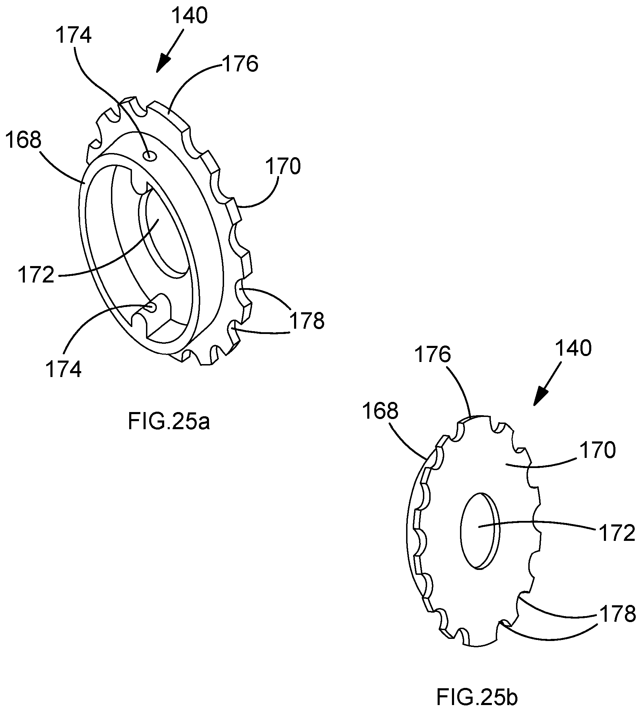

The end cap 140 of the transducer housing is shown in Figure 25. The end cap 140 is generally cylindrical in shape, having a first end 168 and a second end 170. A hollow passage 172 is provided which extends through the end cap 140 between the two ends 168, 170.

The first end 168 of the end cap comprises holes 174 for receiving screws 162. Two holes 174 are provided, although any other suitable number of screw holes could also be used. The number of screw holes 174 present on the end cap 140 is the same as the number of screw holes 160 provided on the small end 154 of the main body 138 of the housing. The screw holes 174 are equally spaced about the circumference of the end cap 140. As can be seen in Figure 25a the two holes 174 are positioned substantially opposite each other. The screw holes 174 on the end cap 140 are configured to line up with the screw holes 160 on the main body 138 of the transducer housing so that these two components can be connected together using screws 162.

The second end 170 of the cap comprises a flange 176. The flange 176 extends radially away from the end cap 140. The flange 176 has an outer perimeter which comprises a plurality of grooves 178. The grooved pattern 178 on the perimeter of the end cap 140 is configured to correspond to the grooved pattern 158 on the small end 154 of the main body 138 of the transducer housing 8. Thus, when the end cap 140 has been connected to the main body 138, the grooved patterns 158, 178 on the two components will align.

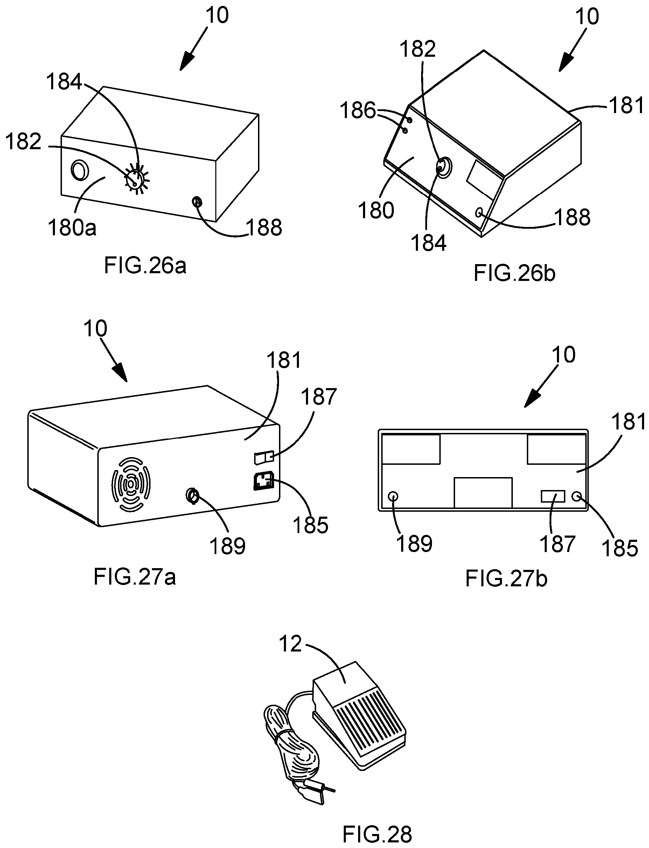

As mentioned previously, the ultrasound generator unit 10, or control box 10, vibrates the needle 4. Referring to Figure 26, the control box 10, or generator unit 10, is substantially box shaped. The control box 10 comprises a sloping control panel 180. That is, the control box 10 has a front face 180 that is slanted backwards, so that a top edge of the front face 180 is tilted towards a rear face 181 of the control box 10. However, as will be understood, in other embodiments the front face 180 may not be sloping. The front face 180 comprises an amplitude control dial 182. The control dial 182 comprises an embedded LED 184, which is used to indicate whether or not the ultrasound is on. The front face 180 also includes a plurality of other LEDs 186 which are used to indicate the status of the generator unit 10. For example, the LEDs 186 may be used to indicate whether the power is on or whether there is a fault. Additionally, the front face 180 comprises a transducer connector 188. This is used to connect the transducer 112 to the control box 10.

Referring to Figure 27, the rear face 181 of the control box 10 comprises a plurality of switches and connectors including a power supply connector and a foot switch connector. There may also be a rocker switch present. The foot switch connector is used to connect the foot switch 12 to the control box 10.

The generator unit 10 is a dedicated adaptable derive electronic control box which is able to track the frequency and vibrational amplitude of the needle. The generator unit 10 is used to vibrate the needle 4 at its resonant frequency. The generator unit 10, or control unit 10, monitors changes in transducer drive frequency and electrical impedance and adapts to the changing conditions in real time. This is done by tuning the drive function accordingly so that the vibration amplitude is maintained at all times.

The control dial 182, or power regulator dial 182, allows the user to control the power, or vibration amplitude, according to the user's requirements. For example the user may wish to reduce the force or increase visibility. The provision of a foot switch connection allows the user to activate the ultrasonics with the press of the pedal 12.

A standard foot pedal activation switch 12 is connected to the control unit 10, or ultrasound generator unit 10, to allow the user to activate the generator unit 10 as and when is needed. The foot switch 12 is connected to the control unit 10 using a standard USB connection as shown in Figure 28, although any other suitable connection can also be used. The control unit 10, once switched on, will be on standby mode until the foot pedal switch 12 is pressed. The needle device 2 will be operational continuously for 5 minutes after which the power generator unit 10 will automatically switch to standby mode.

As already mentioned, the generator unit 10 uses ultrasound energy to vibrate the stylet 14 of the needle device 2. Typically, the stylet 14 is vibrated at a frequency of between 20 - 70 kHz, such as 30 kHz - 60 kHz or 40 - 50 kHz with an amplitude ≤ 2 µm. The stylet 14 of the needle 4 vibrates in a longitudinal, or length-wise, direction. This reduces the penetration force require to insert the needle 4 into the tissue and so the needle's 4 journey into the target is smoother. Thus, the use of longitudinal vibration provide improved cutting of the tissue. The needle-transducer connection therefore plays an important role in ensuring efficient energy transfer from the ultrasound transducer 112 to the needle 4. The stylet 14 is connected to the transducer 112 using a stylet hub 190, or transducer adapter 190.

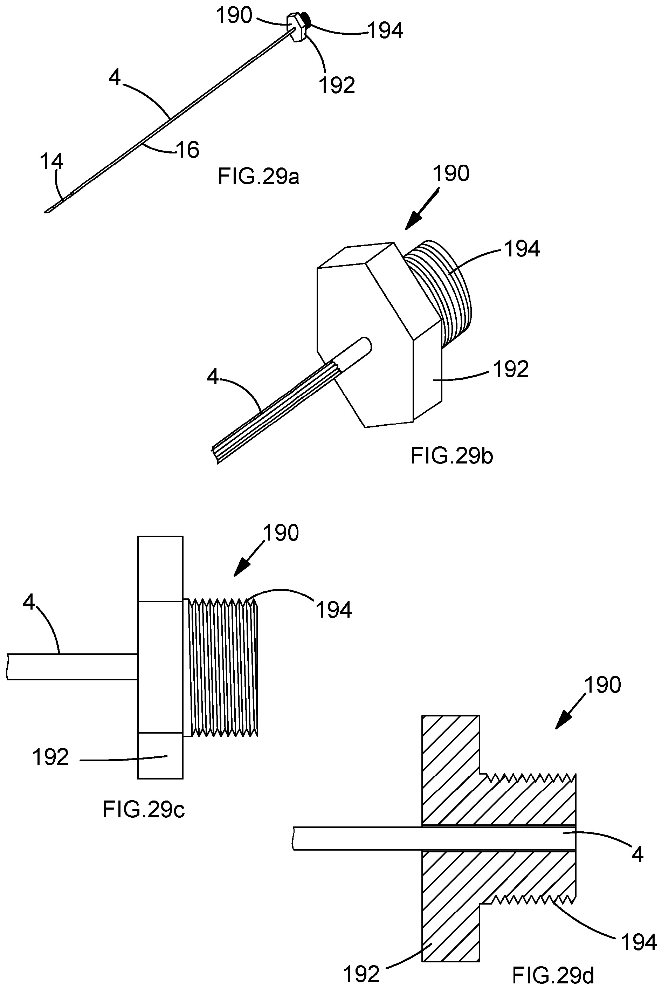

Referring to Figure 29a and 29b, the stylet hub 190 comprises a hexagonally shaped base portion 192 and an extending threaded portion 194. The stylet 14 is attached to the hub 190 on one side of the base portion 192. The base portion has a hole extending through the base portion, partly into the extended threaded portion 194, as shown in Figure 29d. The stylet 14 is inserted into the hole before being attached to the base of the hub 190. Once one end of the stylet has been fully inserted into the hole, the stylet 14 is attached to the base 192 of the stylet hub 190 via a brazed joint. However, any other suitable join may be used, for example the stylet 14 could be laser welded to the base of the hub 192. Inserting the stylet into the hole before the joining process provides a more secure connection between the stylet and the hub.

The extending threaded portion 194 is substantially opposite the stylet joint, as shown in figure 29c. The extending threaded portion 194 is configured to be attached to the front end 120 of the transducer 112 by screwing the stylet hub 190 into the transducer 112.

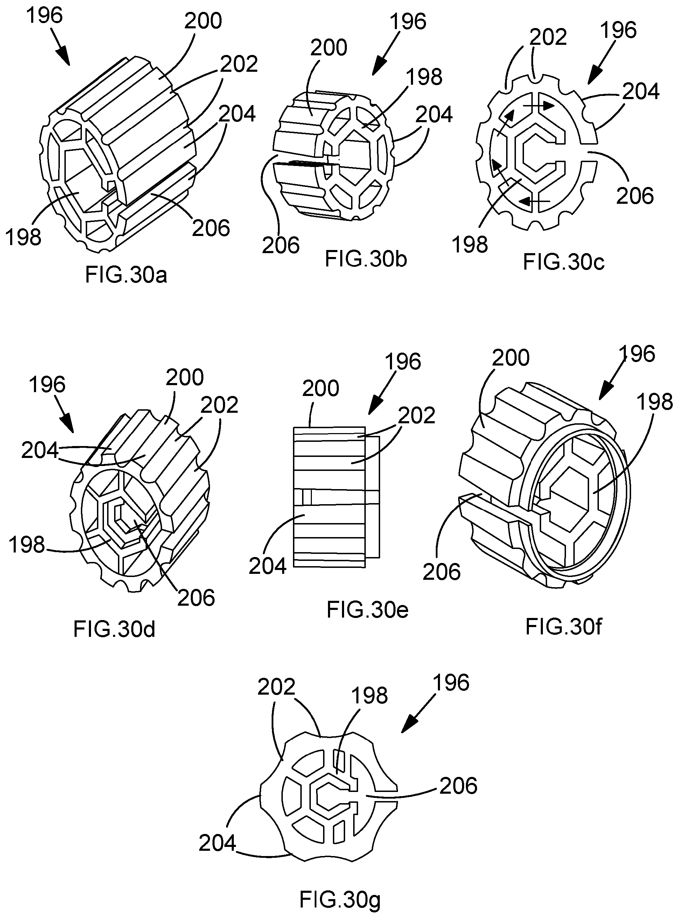

A locking nut 196 may be provided to help the user connect the stylet 14 to the transducer 112. The locking nut 196 is substantially cylindrical in shape, as shown in Figure 30. Inside the cylinder is a hex-shaped socket 198 which extends throughout the length of the locking nut 196, as shown in Figures 30b and 30c. The hex-shaped socket 198 is configured to correspond to the base portion 192 of the stylet hub 190 so that the socket 198 can be fitted around the base of the stylet hub 192.

The external surface of the locking nut 196 is covered in a grippable outer surface 200. The grippable outer surface 200 comprises a plurality of equally spaced apart longitudinal grooves 202 and projections 204. The grippable outer surface 200 helps the user to screw the needle 4 into the transducer's front section 120.

Referring to Figures 30b, 30c, and 30f, a longitudinal slot 206 extends along the entire length of the locking nut 196, passing through the outer surface of the locking nut 196 and the hex socket 198. The slot 206 allows the locking nut 196 to be positioned around the needle 4 to surround the needle 4 during the attachment process and then removed once the needle 4 has been attached to the transducer 112. Although a hex-shaped hub 192 and socket 198 has been described, it will be appreciated that any other suitable shape could be used.

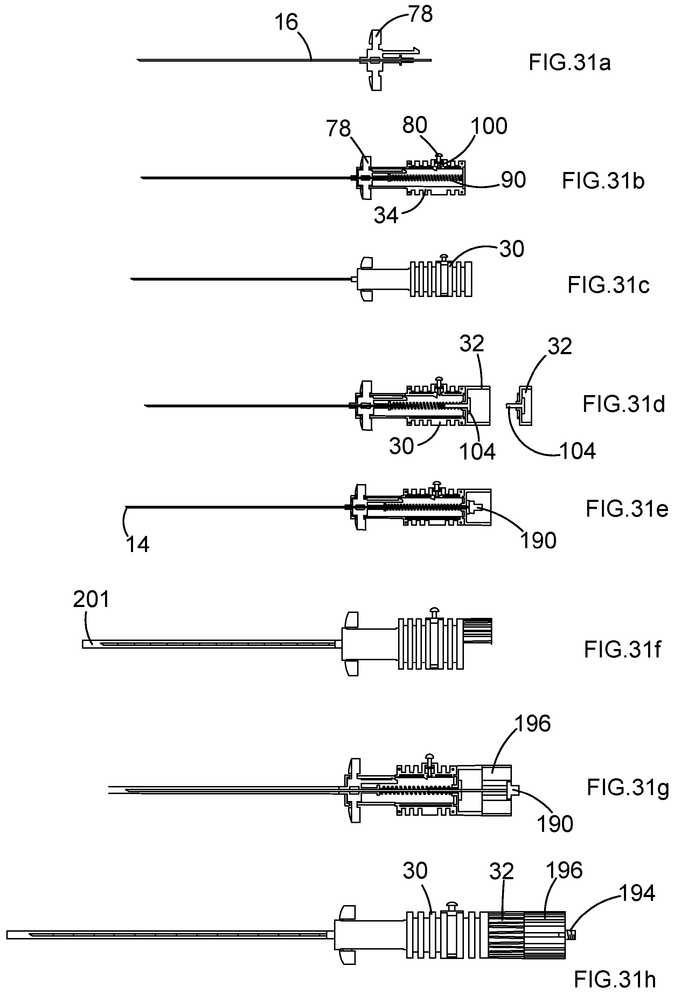

Figure 31 illustrates how to connect the needle 4 to the needle housing 6. Firstly, the cannula 16 is connected to the trigger lever 78, as shown in Figure 31a. This can be done using epoxy, or any other suitable material. The trigger lever 78, trigger button 80, and primary 90 and secondary springs 100 are then placed inside the first shell 34 of the main body 30 of the housing, as shown in Figure 31b. The second shell 36 of the main housing body 30 can then be joined to the first shell 34 using an ultrasonic weld, as shown in figure 31c.

The second spring support 104 is then inserted into the housing cap 32. The housing cap 32 and spring support 104 can then be connected to the main body 30 of the housing by inserting the spring support 104 through the free end of the primary spring 90 and joining the cap 32 to the main body 30 of the housing using ultrasonic welding, as shown in Figure 31d.

The stylet 14, attached to the stylet hub 190, is then inserted through the housing cap 32 and spring support 104, through the primary spring 90 in the needle housing 6, through the trigger level 78 and cannula 16, and extends out through the front end 46 of the needle housing, as shown in Figure 31e.

A needle cover can then be slid over the needle 4, including the cannula 16 and stylet 14, to protect the needle 4 when the device 2 is not being used, as shown in Figure 31f.

The locking nut 196 can then be attached, as shown in figure 31g. To attach the locking nut 196, the stylet hub 190 is pulled back through the end cap 32 until the needle 4 can pass through the slot 206 in the locking nut 196. The hexagonal base 192 of the stylet hub 190 rests inside the hexagonal socket 198 of the locking nut 196 while the threaded portion 194 of the stylet hub 190 extends from the locking nut 196, as can be seen in Figure 31h.

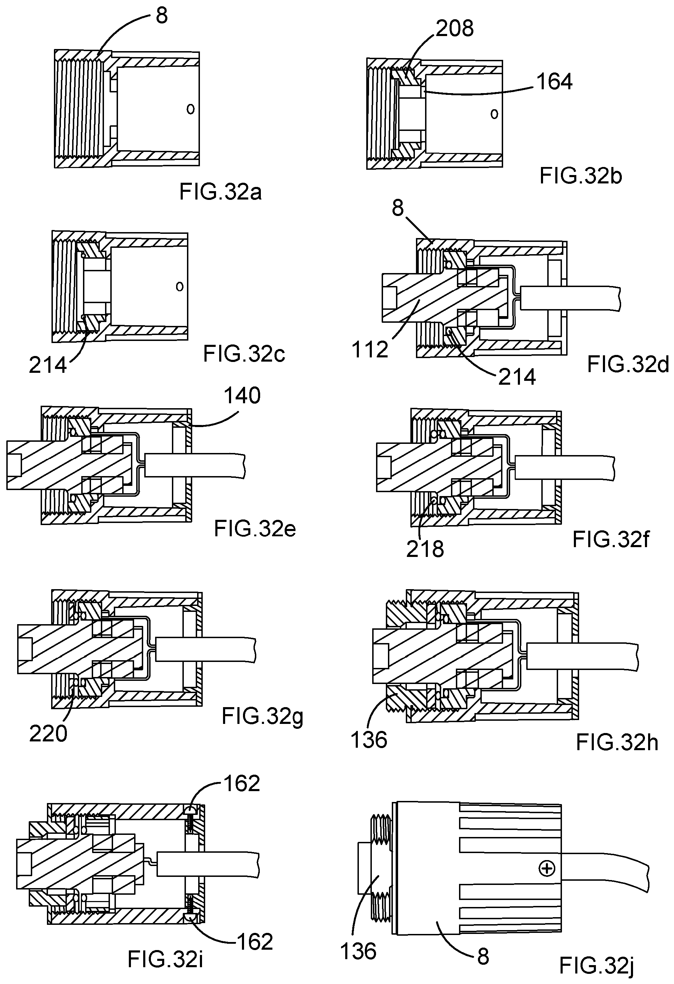

Figure 32 illustrates how to connect the transducer 112 to the transducer housing 8. Firstly a back spacer 208 is inserted into the large end 152 of the main body 138 of the transducer housing until the back spacer 208 abuts the flange 164, as shown in Figure 32b. The flange 164 comprises anti-rotation pins 210 which correspond with anti-rotation slots 212 on the back spacer 208. The anti-rotation pins 210 are inserted into the anti-rotation slots 212. The back space 208 comprises a groove for an O-ring 214. An O-ring 214 is then inserted into the large end 152 of the transducer main body until it fits snugly into the O-ring groove, as shown in Figure 32c. The O-ring 214 ensures that the transducer 112 is sealed tightly in the housing 8.

A coaxial cable 216, attached to the transducer 112, is then passed through the flange 164 and main body 138 of the transducer housing so that the transducer 112 rests on the O-ring 214 inside the housing 8, as shown in Figure 32d. The coaxial cable 216 extends from the small end 154 of the transducer housing 8. The transducer housing cap 140 is then inserted over the coaxial cable 216 to abut the main body 138 of the housing, as shown in Figure 32e.

A second O-ring 218 is then inserted into the large end 152 of the transducer housing 8 so that the flange 122 of the transducer 112 is sandwiched between the two O-rings 214, 218, as shown in Figure 32f. A front spacer 220 is then inserted into the large end 152 of the housing 8, abutting the second O-ring 218, as shown in Figure 32g.

The spacers 208, 220 ensure that the transducer 112 is positioned in the required axial position. The integrated anti-rotational features in the spacers help prevent rotation of the transducer 112 within the transducer housing 8. The integration of the spacers within the housing permits flexibility in the design of the transducer, allowing the housing to accommodate revised transducers that may be required for difference needle gauges and lengths.

The front section 136 of the transducer housing 8 is then screwed into the large end 152 of the transducer housing body 138. The front section 152 is screwed tight enough that the transducer 112 is properly secured inside the housing 8, as shown in Figure 32h.

Once the transducer 112 is secured in place, the end cap 140 of the transducer housing 8 is secured to the main transducer body 138 using screws 162, as shown in Figure 32i. The screws 162 are inserted into the screw holes in the end cap and main body of the housing. The screws 162 are self-tapping screws.

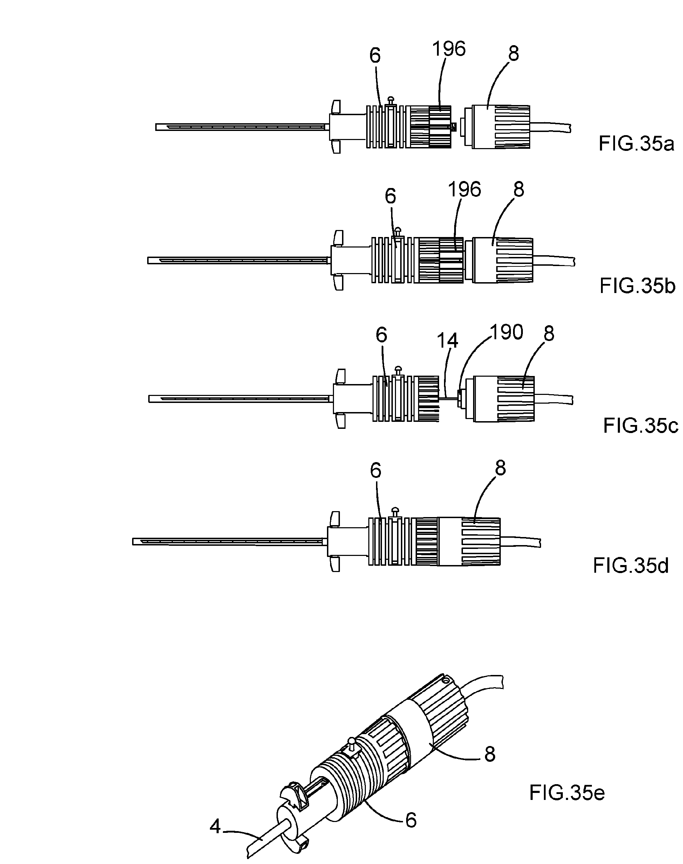

Once the needle housing parts and transducer housing parts have been assembled, the needle housing 6 is connected to the transducer housing 8. This is illustrated in Figure 35.

Firstly, the front end 136 of the transducer housing is aligned with the rear end of the needle housing 32, as shown in Figure 35a. The stylet hub 190 is then screwed into the front mass 114 of the transducer 112 while the needle housing 6 and locking nut 196 are held together, as shown in Figure 35b.

Once the stylet 14 is attached to the transducer 112, the locking nut 196 is removed from between the transducer and needle housings 6, 8, as shown in Figure 35c. The front section 136 of the transducer housing is then screwed into the end cap 32 of the needle housing, attaching the two housings together, as shown in Figures 35d and 35e.

The coaxial cable 216 is then connected to the ultrasound generator unit 10 and the desired power level, or vibration amplitude, is pre-set. The device 2 is then activated by pressing on the foot switch 12.

The needle 4 of the device 2 is generally only used once, for hygiene reasons, but the transducer 112 can be reused. Thus the needle device 2 comprises a single use part, comprising the needle housing 6, and a reusable part, comprising the transducer housing 8, generator unit 10, and foot switch 12. The reusable part therefore connects to the single use part via a screw mechanism. The locking nut 196, or collar 196, may be an intermediate part which facilitates connection of the single use part with the reusable part. However, the single use part may be connected to the reusable part without the need for a locking nut or collar.

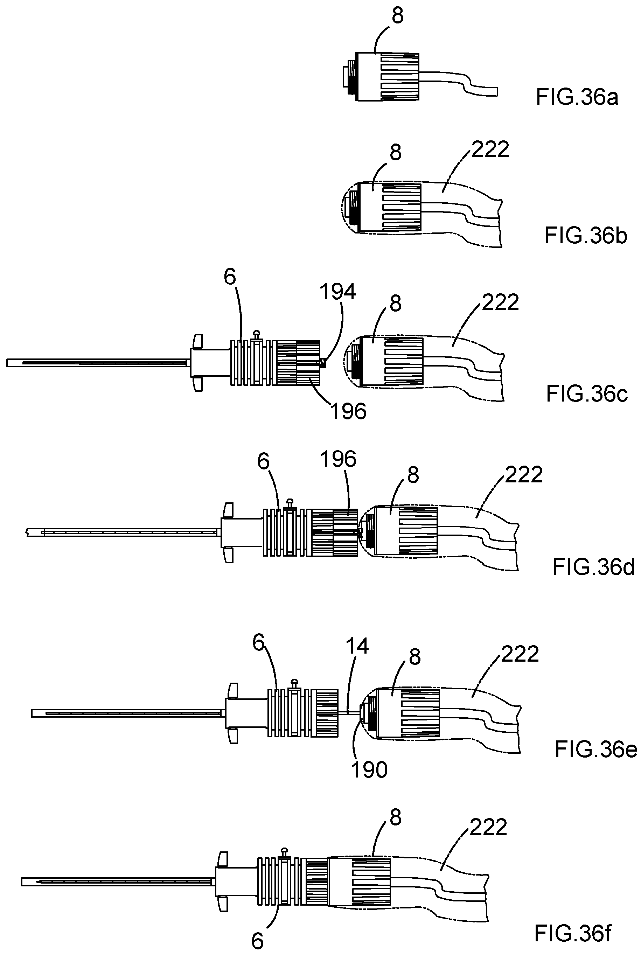

In order for the transducer housing part to be reusable, it should be protected from the single use part using, for example, a sterile protective sheath 222. Figure 36 illustrates how the protective sheath 222 can be used. Firstly, the transducer assembly, including the transducer housing 8 and coaxial cable 216, are wiped using an alcohol wipe, as shown in Figure 36a. The transducer housing 8 is then placed inside a sterile protective sheath 222, or sleeve 222, as shown in Figure 36b. The needle housing 6 is then aligned with the covered transducer housing 8, as shown in Figure 36c. While the needle housing 6 and locking nut 216 are held together, the stylet hub 190 is screwed tightly through the protective sheath 222 and onto the transducer 112, as shown in Figure 36d. The act of screwing the stylet 14 onto the transducer 112 pierces the protective sheath 222. Once the stylet 14 is attached, the locking nut 196 is removed, as shown in Figure 36e. The transducer housing 8 is then screwed onto the needle housing 6, trapping the protective sheath 222 between the two housings, as shown in Figure 36e. The free end of the protective sheath 222, or sleeve 222, can be fixed to the coaxial cable 216 using an elastic band so that the free end does not get in the way of the user.

Once the device 2 has been connected together, it can be used to carry out an ultrasound-guided needle biopsy. An ultrasound probe, not part of and separate to the needle device 2, is used to create an ultrasound image of a region of tissue to be sample. Ultrasonically actuated needles have increased visibility in certain types of medical imaging, such as ultrasound imaging. An oscillating biopsy needle is therefore highly visible under ultrasound and so the location of the needle, in particular the needle tip, can be accurately known.

In addition, vibrating the needle longitudinally at ultrasonic frequencies reduces the penetration force required to introduce the needle 4, namely the stylet 14, into the tissue sample. Thus, the amount by which the needle 4, or stylet 14, deflects upon entry is also reduced.

To vibrate the stylet 14 of the needle 4, the signal generator 10 applies a drive voltage to the piezoelectric rings 130 inside the transducer 112. The amplitude and/or frequency of the drive voltage can be manually adjusted by the user, using the control panel 180 on the generator 10, so the motion of the stylet 14 can be adjusted. The drive voltage applied to the piezoelectric rings 130 causes the piezoelectric rings 130 to be actuated.

Actuation of the piezoelectric rings 130 in the transducer 112 causes reciprocating motion between the front 114 and back 116 masses of the transducer 112. Thus, the relative positions of the front 114 and back 116 masses changes which cause the front mass 114 to move along a longitudinal axis relative to the back mass 116. As the stylet 14 of the needle 4 is connected to the front mass 114 of the transducer 112, via the stylet hub 190, any motion of the front mass 114 is transferred to the stylet. The piezoelectric rings 130 therefore cause the stylet 14 to reciprocate along the longitudinal axis of the needle 4. In other words, the needle 4 is caused to vibrate, by actuating the piezoelectric rings 130 in the transducer 112, with a reciprocating motion along its central longitudinal axis. Only the stylet 14 of the needle 4 is caused to vibrate; the cannula 16 of the needle 4 remains stationary relative to the stylet 14. This is because only the stylet 14 is connected to the transducer 112, via the stylet hub; the cannula 16 is not connected to the transducer 112.

The signal generator can be adjusted to tune the resonant frequency of the piezoelectric rings 130 so that the needle device 2 is optimised for different types of needle stylet 14.

Once the needle 4 is vibrating at the correct frequency and amplitude, the needle 4 is inserted into the tissue. The trigger 78 is then pulled back, withdrawing the cannula 16 and exposing the sample notch 20. The trigger 78 is then released, releasing the cannula 16, to take the biopsy. The needle 3, and its enclosed tissue sample, can then be removed from the body.

Although the needle device 2 has been described using a stylet hub 190 that screws into the transducer 112, other stylet hubs could be used. In some embodiments a gripping device is used to connect the needle to the transducer. An example of a commonly used gripping device includes a collet. A problem with using a gripping device to secure the needle to the transducer is that it is easy to over-tighten or under-tighten the gripping device. If the gripping device is too tight, it may crush the needle. The risk of crushing the needle is especially high if the needle is a hollow needle. If the gripping device is not tight enough then the needle will be loosely connected to the transducer. This may result in inefficient energy transfer between the transducer and the needle. In addition, using a gripping device such as a collet only provides a small point of contact between the needle and transducer. This means that a secure, stable connection is hard to achieve.

In other embodiments, other stylet hubs could be used which avoid the problems associated with the collet style of join. For example, in some embodiments, the stylet hub 190 could be connected to the transducer 112 using a bayonet style connection. In some embodiments a snap-fit connection could be used. Any mechanism which has a large, secure point of contact between the needle and transducer but which does not rely on the provision of compression, or a gripping mechanism, to secure the needle 4 to the transducer 122 would be suitable for use with the needle device 2 described herein.

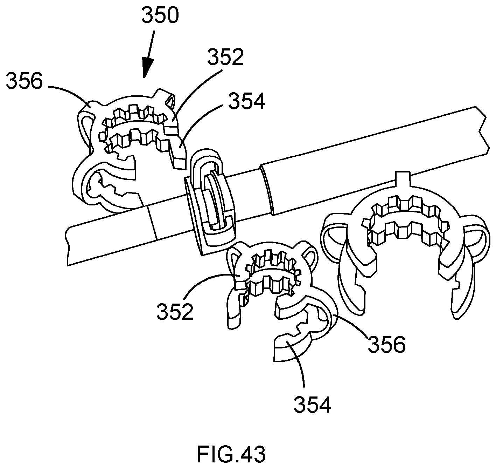

In still further embodiments, the needle can be connected to the transducer using a connection member 350, as shown in Figure 43. The connection member is an external clip 350. The clip comprises two curved arms 352, 354, spaced apart from each other. The arms 352, 354 are connected together by external ribs 356, as shown in Figure 43. One of the arms 354 is configured to be clipped around the external surface of the transducer while the other arm 352 is configured to be clipped around the external surface of the needle. Thus, the clip 350 is configured to maintain the connection between the needle and the transducer.

Although the needle 4 has been described as being single use, in other embodiments the needle 4 can be reused.

Although the vibrating probe has been described with reference to a solid needle, in other embodiments the probe is hollow needle. The hollow needle is used to deliver fluid to body tissues.

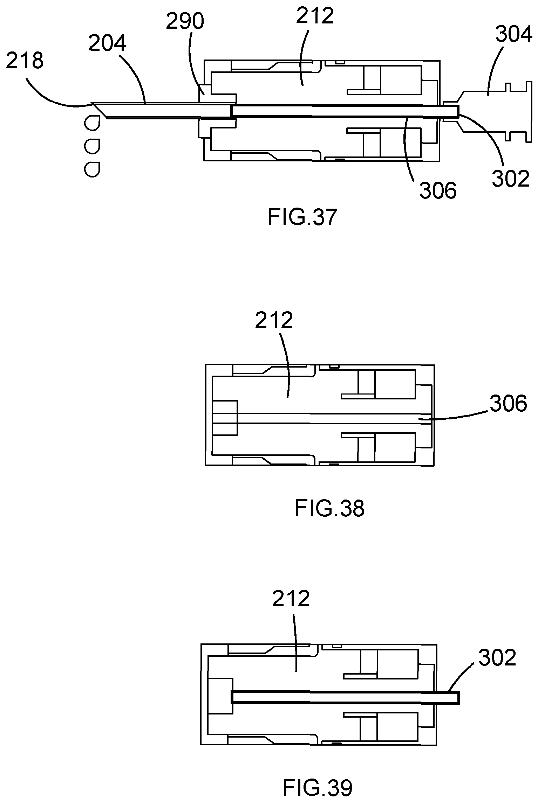

Figure 37 shows an example of a vibrating probe device comprising a hollow needle 204. As before, the needle 204 is connected to one end of the transducer 212 using a hub 290. At the other end of the transducer 212 is a syringe 304. The syringe 304 is connected to the needle 204 using a hollow tube 302. Thus, the tube 302 passes through the centre of the transducer 212.

As before, the transducer 212 is connected to a signal generator (not shown) which allows the transducer to vibrate the needle 204 longitudinally at ultrasonic frequencies. This reduces the penetration force required to insert the needle 2024 into the tissue. Deflection of the needle tip 218 upon entry is also reduced. The syringe 304, after being filled with fluid, is activated by the user so that fluid can be injected into the body.

In order to allow fluid to pass from the syringe 304 at one end of the transducer 212 to the needle 204 at the other end of the transducer 212, the transducer 212 is provided with a channel 306. The channel 306 extends along the entire length of the transducer 212, as can be seen in Figure 38. As well as passing through the main body of the transducer 212, the channel 306 also extends through the pre-stress bolt 232.

In order to provide a sterile environment in which fluid may flow, the hollow tube 302 is inserted into the channel 306 of the transducer 212 before the device is used. The hollow tube is a sterile tube having closed ends at both ends of the tube 302, as shown in Figure 39. This ensures that the inside of the tube 302 remains sealed against potential contaminants when the device is not being used. The closed ends of the sterile tube 302 are penetrated by the syringe 304 and hub 290 when the syringe 304 and needle 204 are connected to the transducer. The device is then ready to be used for fluid injection.

As discussed previously, the pre-stress bolt is needed to maintain tension between the piezoelectric components as well as the front and back masses. Drilling a hole through the bolt 232 to allow the passage of fluid therefore makes the bolt mechanically weak.

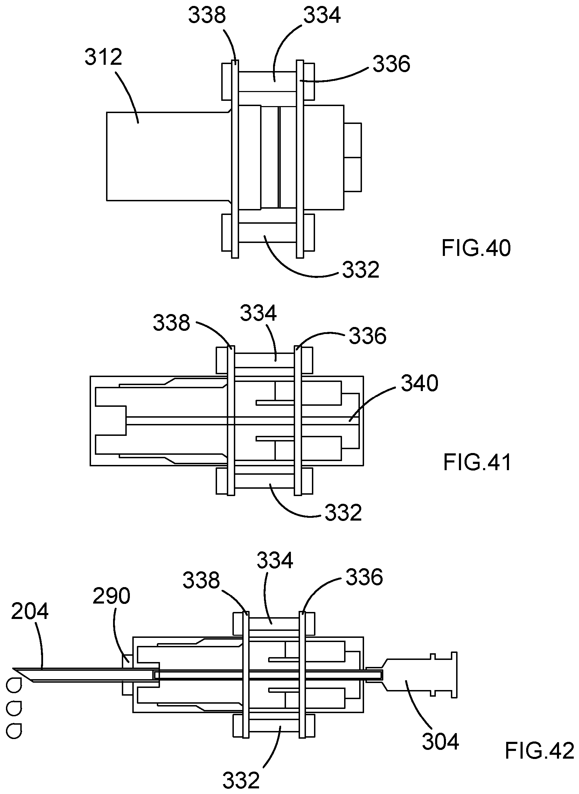

An alternative approach is to provide a transducer 312 having two pre-stress bolts 332, 334, one on either side of the transducer 312, as shown in Figure 40. Each bolt extends longitudinally along a portion of the external surface of the transducer 312. The bolts 332, 334 are spaced apart from each other around the outer perimeter of the transducer 312. As can be seen from Figure 40 and 41, the two bolts are spaced substantially 180° apart from each other.

The bolts are connected to the transducer using two bracer portions 336, 338. The bracer portions 336, 338 are perpendicular to the main body of the transducer 312. The transducer 312 is then provided with a fluid channel 340 passing through the middle of the transducer 312. As before, a hollow tube 302 is inserted into the channel 340 before use and a hollow needle and syringe are connected to either end of the tube 302. The device is then ready to be used to inject fluid into tissue.

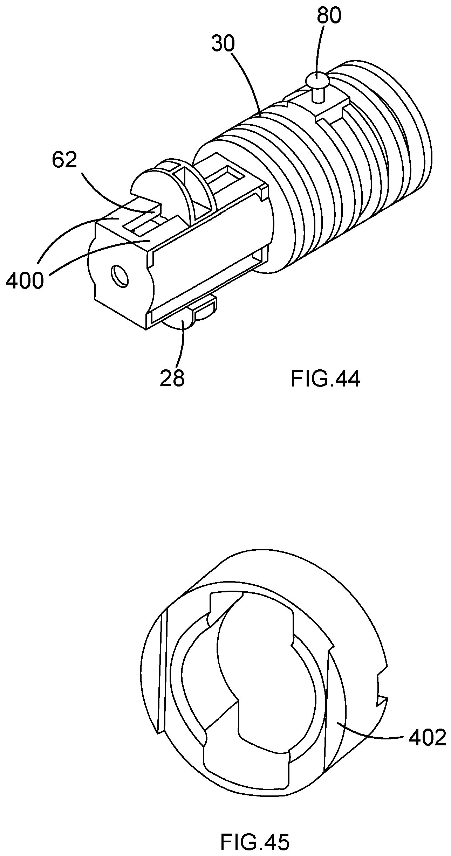

Figure 44 shows an alternative embodiment of a needle housing. The main body 30 has a pair of rails 400 provided on either side on the slots 62 which receive the trigger lever. The rails 400 allow the trigger lever to slide over the needle housing without any lateral motion, or wobble.

Figure 45 shows an alternative embodiment of a back spacer. The anti-rotation pins have been replaced with anti-rotation flats 402.

In some embodiments the catch comprises a barb angle on the flat surface of the catch on both the needle housing and the trigger lever. The engaging surface of the catch and trigger lever may also be rough surface to provide increased friction between the surfaces.

In some embodiments the secondary needle spring positioned around the trigger button may be omitted. In this case, before the trigger has been cocked, the flange of the trigger button will rest the bottom surface of the trigger button slot. When the trigger lever has been cocked, ready for triggering, the engaging surfaces of the trigger button and trigger lever will come into contact with each other. The trigger lever will push up slightly on the trigger button so that the trigger button is raised slightly and protrudes from the trigger button slot, informing the user that the lever has been latched and is ready to be used.

In use, the clinician advances the needle through skin and layers of tissue under ultrasound guidance. Suitably, B-mode (or 2D mode) ultrasound is employed in this context. In B-mode (brightness mode) ultrasound, a linear array of transducers simultaneously scans a plane through the body that can be viewed as a two-dimensional image on screen. The ultrasound beam is fan-shaped, and is positioned over the needle and visualized on the screen. The clinician advances the needle to the target.

The device either has the stylet extended on reaching the target, or it is extended from the cannula on arrival in the location. This is suitably achieved by cocking the device as described above. Sampling occurs when the clinician fires the device and a spring rapidly pushes the outer cannula over the stylet, thereby collection tissue.

The needle is then withdrawn from the patient. Repeating the cocking action of the device reveals a sample of tissue in the sample notch. The sample is then suitably sent for analysis, e.g. pathology.

The device of the invention finds use in a wide variety of clinical procedures. These include, but are not limited to the following:
Amniocentesis - this is a procedure utilized to obtain a sample of amniotic fluid from a pregnant woman's uterus for diagnostic purposes. Such fluid is obtained, by inserting a long spinal needle, having a sharp-cutting tip, through the skin, fascia and uterine muscle into the uterine cavity and obtaining therefrom such amniotic fluid by aspiration. Complications, including trauma, haemorrhage and infection have resulted from employing such prior-art surgical needle in such procedure. The device of the invention, being capable of accurate guidance under ultrasound imaging (a non-invasive imaging technique known to be safe to unborn infants) is advantageous compared to known devices and methods.

Chorionic villus sampling - chorionic villi are finger-like projections of tissue in the chorionic membrane which eventually forms the placenta. Chorionic villi are well developed around the seventh to eighth weeks of pregnancy. The object of this procedure is to remove, by vacuum, a sample of the villi and assay the sample to determine the genetic health of the fetus. A physician inserts a thin catheter (consisting of a cannula containing an obturator) through the vagina and cervix into the uterus ending at the chorion membrane. When the catheter tip is located on the villi, a source of negative pressure is coupled to the catheter to withdraw a sample of villi tissue for analysis. The device of the invention, being capable of accurate guidance under ultrasound imaging (a non-invasive imaging technique known to be safe to unborn infants) is advantageous compared to known devices and methods.

Vacuum-assisted biopsy - through a small incision or cut in the skin, a biopsy needle is inserted into e.g. the breast and, using a vacuum-powered instrument, several tissue samples are taken. The vacuum draws tissue into the centre of the needle and a rotating cutting device takes the samples. The samples are retrieved from the centre of the biopsy needle following the procedure and sent to a laboratory to be examined by a pathologist (a specialist doctor trained in diagnosing biopsies).

The biopsy procedure is performed under imaging guidance (mammogram, magnetic resonance imaging (MRI) or ultrasound). In other words, the pictures or images obtained from scans allow the radiologist performing the biopsy to make sure the needle is correctly positioned. The devices of some embodiments of the invention are advantageous in vacuum-assisted biopsy procedures. Similarly, the devices of the invention are useful in vacuum-assisted excision of tumours (ultrasound-guided vacuum excision, or UGVAE).

*In vitro* fertilization (IVF) - in such procedures, eggs are usually retrieved from the patient by transvaginal oocyte retrieval involving an ultrasound-guided needle piercing the vaginal wall to reach the ovaries. Through this needle, follicles can be aspirated, and the follicular fluid is handed to the IVF laboratory to identify and diagnose the ova. The fertilized egg, (embryo), or usually multiple embryos, are then transferred to the patient's uterus with the intention of establishing a successful pregnancy. The devices of some embodiments of the invention are advantageous in IVF methods, both for egg retrieval and embryo implantation.

Localized drug delivery - frequently, it is desirable to infuse solutions of medicaments to a particular region or organ of the body. Such medicaments include anaesthetics (e.g. for local anaesthesia), particles for embolization (embolotherapy), and nanoparticles. The devices of the invention are useful in this context, as they allow delivery of medicaments to precise locations under ultrasound guidance. In particular, it is advantageous to use the devices of the invention as the action of the needle may improve distribution of drug/liquids or colloids.

Fine-needle aspiration (FNA) - a diagnostic procedure used to investigate lumps or masses. In this technique, a thin, hollow needle is inserted into the mass for sampling of cells that, after being stained, will be examined under a microscope (biopsy). The sampling and biopsy considered together are called fine-needle aspiration biopsy (FNAB) or fine-needle aspiration cytology (FNAC). The ability of the clinician to guide a needle tip to the desired sampling locality under ultrasound guidance provided by the devices of the present invention makes these advantageous in fine-needle aspiration. It is believed that the action of the needle helps to dislodge cells from the target and improve sampling.

Radiofrequency ablation (RFA) - a medical procedure in which part of the electrical conduction system of the heart, tumour or other dysfunctional tissue is ablated using the heat generated from medium frequency alternating current (in the range of 350-500 kHz). RFA is generally conducted in the outpatient setting, using either local anaesthetics or conscious sedation anaesthesia. When it is delivered *via* catheter, it is called radiofrequency catheter ablation. Clearly, it is very desirable in such procedures that the ablation probe is correctly located proximal to the dysfunctional tissue; the devices of the present invention makes these advantageous in such techniques.

Stent placement - insertion of a metal or plastic tube (stent) into the lumen of an anatomic vessel or duct to keep the passageway open. Included are expandable coronary, vascular and biliary stents. The devices of the present invention are advantageous in the accurate placement of stents, as they are capable of guidance under ultrasound visualisation.

## Claims

1. A device (2) for use in a medical procedure, the device comprising:
an elongate member having a first end, a second end, and a longitudinal axis extending between said ends, said first end being a sharps end, and said second end comprising an integral hub;
an ultrasonic transducer (112) comprising a socket adapted to receive said hub;
**characterised by** the transducer (112) being configured to oscillate the elongate member substantially along the longitudinal axis at a frequency of above 20 kHz; and
wherein the maximum amplitude of oscillation is limited to ≤2 µm.

2. A device according to claim 1 wherein the ultrasonic transducer (112) is configured to oscillate the elongate member at a frequency of between 20 kHz and 70 kHz, preferably 40 kHz and 50 kHz.

3. A device according to any preceding claim wherein the force required to advance the first end of the elongate member through body tissue is reduced when the elongate member is oscillated by the transducer (112) relative to when it is not oscillated.

4. A device according to any preceding claim wherein the hub comprises an externally threaded portion (144); and/or
wherein the socket comprises an internally threaded portion; and/or
wherein the hub and socket form a bayonet connection; or
wherein the hub and socket form a snap-fit connection.

5. A device according to any of the preceding claims wherein the elongate member is a needle (4).

6. A device according to claim 5, wherein the needle (4) is a hollow needle;
wherein the hollow needle comprises a passage configured to allow fluid to pass through the needle (4);
wherein the transducer (112) comprises a channel configured to allow fluid to pass through the transducer (112);
wherein the channel is adapted to receive a sterile tube; and
wherein the hollow needle is adapted to receive a first end of the sterile tube.

7. A device as claimed in any preceding claim wherein the elongate member is a stylet comprising a sample notch towards the sharps end.

8. A device as claimed in claim 7, further comprising a cannula (16) at least partially enclosing the stylet, and slidable relative to the stylet along its longitudinal axis;
wherein the cannula (16) comprises a cutting tip which is symmetric about a central longitudinal axis of the cannula (16);
wherein in a first configuration the sample notch is concealed within the cannula (16), and a second configuration in which the cannula (16) is withdrawn along the longitudinal axis of the stylet sufficient to expose the sample notch;
the device further comprising a spring loading mechanism adapted to advance the cannula (16) along the longitudinal axis of the stylet from the second configuration to the first configuration.

9. A device as claimed in claim 8, further comprising a trigger adapted to hold the cannula in the second configuration and release the cannula (16) upon actuation of said trigger by a user causing the cannula (16) to advance rapidly to the first configuration.

10. A device as claimed in any preceding claim, further comprising a locking nut (196).

11. A device as claimed in claim 10, wherein the locking nut (196) comprises a socket portion.

12. A device as claimed in claim 11, wherein an internal surface of the socket portion is shaped to correspond to an external perimeter of the base portion of the elongate member hub.

13. A device as claimed in any one of claims 10 to 12, wherein the base portion of the elongate member hub and the socket portion of the locking nut (196) are configured to releasably engage.

14. A device as claimed in any one of claims 10 to 13, wherein the locking nut (196) comprises an external surface, the external surface comprising a grippable portion.

15. A device as claimed in any of the preceding claims, wherein the amplitude and/or frequency of the oscillations produced by the transducer (112) are controllable by a user.

## Patentansprüche

1. Vorrichtung (2) zur Verwendung in einer medizinischen Prozedur, wobei die Vorrichtung umfasst:
ein längliches Element, das ein erstes Ende, ein zweites Ende und eine Längsachse aufweist, die sich zwischen den Enden erstreckt, wobei das erste Ende ein scharfes Ende ist und das zweite Ende eine integrierte Nabe umfasst;
einen Ultraschallwandler (112), der eine Buchse umfasst, die zur Aufnahme der Nabe angepasst ist;
**dadurch gekennzeichnet, dass** der Wandler (112) so konfiguriert ist, dass er das längliche Element im Wesentlichen entlang der Längsachse mit einer Frequenz von über 20 kHz in Schwingung versetzt; und
wobei die maximale Schwingungsamplitude auf ≤2 µm begrenzt ist.

2. Vorrichtung nach Anspruch 1, wobei der Ultraschallwandler (112) so konfiguriert ist, dass er das längliche Element mit einer Frequenz zwischen 20 kHz und 70 kHz, vorzugsweise 40 kHz und 50 kHz, in Schwingung versetzt.

3. Vorrichtung nach einem vorstehenden Anspruch, wobei die zum Vorschieben des ersten Endes des länglichen Elements durch Körpergewebe erforderliche Kraft, wenn das längliche Element von dem Wandler (112) in Schwingung versetzt wird, im Vergleich dazu verringert ist, wenn es nicht in Schwingung versetzt wird.

4. Vorrichtung nach einem vorstehenden Anspruch, wobei die Nabe einen Abschnitt mit Außengewinde (144) umfasst; und/oder
wobei die Buchse einen Abschnitt mit Innengewinde umfasst; und/oder
wobei die Nabe und Buchse eine Bajonettverbindung bilden; oder
wobei die Nabe und Buchse eine Schnappverbindung bilden.

5. Vorrichtung nach einem der vorstehenden Ansprüche, wobei das längliche Element eine Nadel (4) ist.

6. Vorrichtung nach Anspruch 5, wobei die Nadel (4) eine Hohlnadel ist;
wobei die Hohlnadel einen Durchgang umfasst, der so konfiguriert ist, dass Flüssigkeit durch die Nadel (4) fließen kann;
wobei der Wandler (112) einen Kanal umfasst, der so konfiguriert ist, dass Flüssigkeit durch den Wandler (112) fließen kann;
wobei der Kanal zum Aufnehmen eines sterilen Schlauches angepasst ist; und
wobei die Hohlnadel zum Aufnehmen eines ersten Endes des sterilen Schlauchs angepasst ist.

7. Vorrichtung nach einem vorstehenden Anspruch, wobei das längliche Element ein Stilett ist, das am spitzen Ende eine Probenkerbe umfasst.

8. Vorrichtung nach Anspruch 7, die weiter eine Kanüle (16) umfasst, die das Stilett zumindest teilweise umschließt und relativ zum Stilett entlang seiner Längsachse verschiebbar ist;
wobei die Kanüle (16) eine Schneidspitze umfasst, die symmetrisch um eine zentrale Längsachse der Kanüle (16) ist;
wobei in einer ersten Konfiguration die Probenkerbe innerhalb der Kanüle (16) verborgen ist, und in einer zweiten Konfiguration die Kanüle (16) entlang der Längsachse des Stiletts weit genug zurückgezogen ist, um die Probenkerbe freizulegen;
wobei die Vorrichtung weiter einen Federbelastungsmechanismus umfasst, der dazu angepasst ist, die Kanüle (16) entlang der Längsachse des Stiletts aus der zweiten Konfiguration in die erste Konfiguration vorzuschieben.

9. Vorrichtung nach Anspruch 8, die weiter einen Auslöser umfasst, der dazu angepasst ist, die Kanüle in der zweiten Konfiguration zu halten und die Kanüle (16) freizugeben, wenn ein Benutzer den Auslöser betätigt, wodurch bewirkt wird, dass die Kanüle (16) schnell in die erste Konfiguration vorgeschoben wird.

10. Vorrichtung nach einem vorstehenden Anspruch, die weiter eine Sicherungsmutter (196) umfasst.

11. Vorrichtung nach Anspruch 10, wobei die Sicherungsmutter (196) einen Buchsenabschnitt umfasst.

12. Vorrichtung nach Anspruch 11, wobei eine Innenfläche des Buchsenabschnitts so geformt ist, dass sie einem Außenumfang des Basisabschnitts der Nabe des länglichen Elements entspricht.

13. Vorrichtung nach einem der Ansprüche 10 bis 12, wobei der Basisabschnitt der Nabe des länglichen Elements und der Buchsenabschnitt der Sicherungsmutter (196) so konfiguriert sind, dass sie lösbar ineinandergreifen.

14. Vorrichtung nach einem der Ansprüche 10 bis 13, wobei die Sicherungsmutter (196) eine Außenfläche umfasst, wobei die Außenfläche einen greifbaren Abschnitt umfasst.

15. Vorrichtung nach einem der vorstehenden Ansprüche, wobei die Amplitude und/oder Frequenz der vom Wandler (112) erzeugten Schwingungen von einem Benutzer steuerbar sind.

## Revendications

1. Dispositif (2) pour une utilisation dans une procédure médicale, le dispositif comprenant :
un élément allongé présentant une première extrémité, une seconde extrémité et un axe longitudinal s'étendant entre lesdites extrémités, ladite première extrémité étant une extrémité pointue et ladite seconde extrémité comprenant un moyeu intégré ;
un transducteur à ultrasons (112) comprenant une douille adaptée pour recevoir ledit moyeu ;
**caractérisé par** le transducteur (112) étant configuré pour faire osciller l'élément allongé sensiblement le long de l'axe longitudinal à une fréquence supérieure à 20 kHz ; et
dans lequel l'amplitude maximale d'oscillation est limitée à ≤ 2 µm.

2. Dispositif selon la revendication 1, dans lequel le transducteur à ultrasons (112) est configuré pour faire osciller l'élément allongé à une fréquence comprise entre 20 kHz et 70 kHz, de préférence 40 kHz et 50 kHz.

3. Dispositif selon une quelconque revendication précédente, dans lequel la force requise pour faire avancer la première extrémité de l'élément allongé à travers le tissu corporel est réduite lorsque l'élément allongé est mis en oscillation par le transducteur (112) par rapport au moment où il n'est pas mis en oscillation.

4. Dispositif selon une quelconque revendication précédente, dans lequel le moyeu comprend une partie filetée extérieurement (144) ; et/ou
dans lequel la douille comprend une partie filetée intérieurement ; et/ou
dans lequel le moyeu et la douille forment une connexion à baïonnette ; ou
dans lequel le moyeu et la douille forment une connexion à encliquetage.

5. Dispositif selon l'une quelconque des revendications précédentes, dans lequel l'élément allongé est une aiguille (4).

6. Dispositif selon la revendication 5, dans lequel l'aiguille (4) est une aiguille creuse ;
dans lequel l'aiguille creuse comprend un passage configuré pour permettre à un fluide de passer à travers l'aiguille (4) ;
dans lequel le transducteur (112) comprend un canal configuré pour permettre à un fluide de passer à travers le transducteur (112) ;
dans lequel le canal est adapté pour recevoir un tube stérile ; et
dans lequel l'aiguille creuse est adaptée pour recevoir une première extrémité du tube stérile.

7. Dispositif selon une quelconque revendication précédente, dans lequel l'élément allongé est un stylet comprenant une encoche d'échantillon vers l'extrémité pointue.

8. Dispositif selon la revendication 7, comprenant en outre une canule (16) entourant au moins partiellement le stylet et pouvant coulisser par rapport au stylet le long de son axe longitudinal ;
dans lequel la canule (16) comprend une pointe coupante qui est symétrique autour d'un axe longitudinal central de la canule (16) ;
dans lequel, dans une première configuration, l'encoche d'échantillon est dissimulée dans la canule (16) et une seconde configuration dans laquelle la canule (16) est retirée le long de l'axe longitudinal du stylet suffisamment pour exposer l'encoche d'échantillon ;
le dispositif comprenant en outre un mécanisme de chargement à ressort adapté pour faire avancer la canule (16) le long de l'axe longitudinal du stylet de la seconde configuration à la première configuration.

9. Dispositif selon la revendication 8, comprenant en outre une gâchette adaptée pour maintenir la canule dans la seconde configuration et libérer la canule (16) lors de l'actionnement de ladite gâchette par un utilisateur, provoquant l'avancée rapide de la canule (16) vers la première configuration.

10. Dispositif selon une quelconque revendication précédente, comprenant en outre un écrou de verrouillage (196).

11. Dispositif selon la revendication 10, dans lequel l'écrou de verrouillage (196) comprend une partie de douille.

12. Dispositif selon la revendication 11, dans lequel une surface interne de la partie de douille est formée pour correspondre à un périmètre externe de la partie de base du moyeu de l'élément allongé.

13. Dispositif selon l'une quelconque des revendications 10 à 12, dans lequel la partie de base du moyeu de l'élément allongé et la partie de douille de l'écrou de verrouillage (196) sont configurées pour venir en prise de manière amovible.

14. Dispositif selon l'une quelconque des revendications 10 à 13, dans lequel l'écrou de verrouillage (196) comprend une surface externe, la surface externe comprenant une partie pouvant être saisie.

15. Dispositif selon l'une quelconque des revendications précédentes, dans lequel l'amplitude et/ou la fréquence des oscillations produites par le transducteur (112) peuvent être régulées par un utilisateur.
